# EUROPEAN PATENT SPECIFICATION

(11) **EP 2 950 816 B1**
(45) Date of publication and mention of the grant of the patent: **31.07.2019**
(21) Application number: 14702282.6
(22) Date of filing: 04.02.2014
(51) Int. Cl.: A61K 39/12, A61K 39/39, A61P 31/14

(54) **THE USE OF DNA SEQUENCES ENCODING AN INTERFERON AS VACCINE ADJUVANTS**
VERWENDUNG VON DNA-SEQUENZEN ZUR CODIERUNG FÜR EIN INTERFERON ALS IMPFSTOFF-ADJUVANS
UTILISATION DE SÉQUENCES D'ADN CODANT POUR UN INTERFÉRON EN TANT QU'ADJUVANTS DE VACCIN

(30) Priority: 04.02.2013 NO 20130175
(43) Date of publication of application: 09.12.2015
(73) Proprietor: Schweitzer Biotech Company Ltd., Taipei, 11493 (TW)
(72) Inventor: ROBERTSEN, Børre., 9007 Tromsø (NO); CHANG, Chia Jung., 9037 Tromsø (NO)
(74) Representative: Onsagers AS
(86) International application number: PCT/EP2014/052111
(87) International publication number: WO 2014/118385

(56) References cited:
- WO-A2-02/060921
- WO-A2-2006/099451
- ANDERSON D P: "IMMUNOSTIMULANTS, ADJUVANTS, AND VACCINE CARRIERS IN FISH: APPLICATIONS TO AQUACULTURE", ANNUAL REVIEW OF FISH DISEASES, XX, XX, vol. 2, 1 January 1992 (1992-01-01), pages 281-307, XP008038231, ISSN: 0959-8030, DOI: 10.1016/0959-8030(92)90067-8
- SVINGERUD T ET AL: "Atlantic salmon type I IFN subtypes show differences in antiviral activity and cell-dependent expression: Evidence for high IFNb/IFNc-producing cells in fish lymphoid tissues", THE JOURNAL OF IMMUNOLOGY, THE AMERICAN ASSOCIATION OF IMMUNOLOGISTS, US, vol. 189, no. 12, 15 December 2012 (2012-12-15), pages 5912-5923, XP002717805, ISSN: 0022-1767, DOI: 10.4049/JIMMUNOL.1201188 [retrieved on 2012-11-19]
- SUN B ET AL: "Identification of an Atlantic salmon IFN multigene cluster encoding three IFN subtypes with very different expression properties", DEVELOPMENTAL AND COMPARATIVE IMMUNOLOGY, PERGAMON PRESS, US, vol. 33, no. 4, 1 April 2009 (2009-04-01), pages 547-558, XP025922907, ISSN: 0145-305X, DOI: 10.1016/J.DCI.2008.10.001 [retrieved on 2008-11-11] cited in the application
- BØRRE ROBERTSEN ET AL: "Atlantic Salmon Interferon Genes: Cloning, Sequence Analysis, Expression, and Biological Activity", JOURNAL OF INTERFERON & CYTOKINE RESEARCH, vol. 23, no. 10, 1 October 2003 (2003-10-01), pages 601-612, XP055113981, ISSN: 1079-9907, DOI: 10.1089/107999003322485107 cited in the application
- BIERING E ET AL: "UPDATE ON VIRAL VACCINES FOR FISH", DEVELOPMENTS IN BIOLOGICALS, KARGER, BASEL, vol. 121, 1 January 2005 (2005-01-01), pages 97-113, XP001207868, ISSN: 1424-6074

## Description

The present invention relates to the use of a nucleic acid sequence encoding a type I interferon (IFN) originating from Atlantic salmon, more specifically IFNa1, IFNa2, IFNb and IFNc, as a vaccine adjuvant useful in the prevention or treatment of an infection by a microbial pathogen or a parasite.

### Background of the invention

Interferons (IFNs) belong to a family of cytokines expressed and secreted by vertebrate cells in response to virus infection. The activity of the secreted IFNs is exerted through binding to IFN receptors on host cells, resulting in a complex change of signaling that activates transcription of IFN stimulated genes (ISGs) some of which encode antiviral proteins [Sadler AJ, Williams BR. Interferon-inducible antiviral effectors. Nat Rev Immunol. 2008 Jul;8(7):559-68].

Interferons have been classified by their chemical and biological characteristics and are typically divided into three classes, i.e. type I IFNs, type II IFNs and type III IFNs [Donnelly RP, Kotenko SV. Interferon-lambda: a new addition to an old family. J Interferon Cytokine Res. 2010 Aug; 30(8):555-64].

Type I IFNs include the mammalian IFN-α and IFN-β, which are induced by viruses in most cells and have a crucial role in the innate immunity against viruses. Type II IFN is identical to IFN-γ and is produced by natural killer cells (NK cells) and T-lymphocytes. IFN-γ plays a central role in the T-cell mediated immune response of the adaptive immune system whereby it activates macrophages for enhanced killing of intracellular bacteria. Type III IFNs have similar roles as type I IFN in innate immunity against viruses, but type I IFN and type III IFNs signal through different receptors, which explains why type I IFNs induce antiviral activity in most cells while type III IFNs mainly induce antiviral activity in cells of epithelial origin [Donnelly RP, Kotenko SV. Interferon-lambda: a new addition to an old family. J Interferon Cytokine Res. 2010 Aug; 30(8):555-64]. Type III IFNs are also distinct from type I IFNs with regard to sequence and gene structure.

Typically, type I IFNs are induced rapidly during virus infection through host cell recognition of virus RNA and are then secreted and transported by the blood stream. The antiviral effect of IFNs is exerted through the binding to the IFN receptor, which is present on most cells [Sadler AJ, Williams BR. Interferon-inducible antiviral effectors. Nat Rev Immunol. 2008 Jul;8(7):559-68)]. Binding triggers signal transduction through the Jak-Stat pathway and leads to transcription of several antiviral genes such as Mx and ISG15 [Sadler AJ, Williams BR. Interferon-inducible antiviral effectors. Nat Rev Immunol. 2008 Jul; 8(7):559-68)].

Characteristic of the mammalian type I IFNs is that they are encoded by intronless genes and bind to the same heterodimeric receptor containing the IFNAR1 and IFNAR2 subunits. Human type I IFNs are encoded by over 20 genes clustered on chromosome 9 encompassing 13 functional IFN-α genes, 1 IFN-β gene, 1IFN-ω gene, 1 IFN-ε, and 1IFN-κ gene [Pestka S, Krause CD, Walter MR. Interferons, interferon-like cytokines, and their receptors. Immunol Rev 2004;202: 8-32].

Although the first human interferon gene was cloned as early as in 1980 (Tanigushi et al, Nature, 1980, 285(5766), pp. 547-549, Taniguchi et al, Proc Natl Acad Sci, 1980, 77(7), pp. 4003-4006), the first IFN coding genes in fish were isolated in 2003 from zebrafish, Atlantic salmon and the pufferfish *Tetraodon nigrovirides*, respectively (Altman et al., J Virol, 2003, 77(3), pp. 1992-2002; Robertsen et al., J Interferon Cytokin Res, 2003, 23 (10), pp. 601-612; Robertsen, Norwegian patent application NO 20033000; Lutfalla et al., BMC Genomics 2003, 4(1), pp. 29). Thereafter, IFN genes were also identified in other species; e.g. channel catfish (Long et al, Dev. Comp Immunol, 2004, 28(2), pp. 97-111) and sea bass (Casani et al., Mol. Immunol., 2009, 46, pp. 943 - 952).

WO201101760 disclose a set of interferon polypeptides and genes of human origin. There is not experimental data provided in WO201101760 that may show that the interferon polypeptides or genes encoding said polypeptides are useful as vaccine adjuvants.

The type I IFNs identified in fish differ from the human type I IFNs in that the fish IFN genes contain introns. Moreover, fish IFNs show very low sequence similarity to mammalian IFNs (Robertsen , Fish Shellfish Immunol, 2006, 20, pp. 172-191). Later research has furthermore revealed that the type I IFN family in fish is quite larger than originally assumed. Today, 4 different subtypes of type I IFNs, denoted IFNa, IFNb, IFNc and IFNd, have been identified in Atlantic salmon and rainbow trout, respectively (Sun et al., Dev. Comp.Immunol., 2009, 33, pp. 547 - 558, Chang et al., Immunogenetics, 2009, 61, pp. 315-325). The largest cluster of IFN genes has been found in Atlantic salmon, where eleven IFN genes were identified in the same genomic region encoding two IFNa (IFNal and IFNa3), four IFNb (IFNb1-b4) and five IFNc (IFNc1-c4) genes (Sun et al., 2009, *supra*). IFNc homologs have been identified in Atlantic salmon and zebrafish, but not in rainbow trout (Chang et al., 2009, *supra*, Zou et al., J. Immunol., 2007, 179, pp. 3859 - 3871, Purcell et al., Fish Shellfish Immunol., 2009, 26, pp. 293 - 304). Salmon IFNa2 (Robertsen B, Bergan V, Rokenes T, Larsen R, Albuquerque A. Atlantic salmon interferon genes: cloning, sequence analysis, expression, and biological activity. J Interferon Cytokine Res 2003;23: 601-612; Bergan V, Steinsvik S, Xu H, Kileng O, Robertsen B. Promoters of type I interferon genes from Atlantic salmon contain two main regulatory regions. FEBS J 2006;273: 3893-3906.) and IFNd (Gene bank accession no. AGKD01088706) are encoded by single genes outside of this cluster.

Fish type I IFNs may be further divided into two groups based on whether they contain two or four disulfide bridging cysteines (Zou et al., 2009, *supra*, Hamming et al., J. Virol., 2011, J. Virol., 85, pp. 8181-8187). IFNa and IFNd have been found to comprise two cysteines, and IFNc and IFNb four cysteines (Hamming et al., 2011, *supra*, Zou et al., 2009, *supra*).

Interestingly, Atlantic salmon IFNa, IFNb, IFNc (Sun et al., 2009, *supra*) and IFNd subtypes have only 25 to 37 % amino acid sequence identity between themselves.

Comparisons of the mammalian IFNs and fish IFNs by sequence analysis have revealed that fish and mammalian IFNs have followed different evolutionary routes (Robertsen, Fish and Shellfish Immunology,2008, 25, pp 351-357). Mammalian IFN-α and IFN-β do thus not have true homologs in fish. Moreover, it has recently been established that zebrafish type I IFNs possessing 2 cysteines bind to a different receptor than IFNs with 4 cysteines (Aggad et al., J. Immunol., 2009, 183, pp3924-3931). This is different from mammals in which all type I IFNs bind to the same receptor. Thus, even though one might point to some similarities between mammalian and fish IFNs, the differences render it difficult to predict the various roles of type I IFNs in fish immunology and viral defence.

Antiviral activity of IFNa homologs has been shown in several fish species including Atlantic salmon (Zou et al., Dev. Comp. Immunol. 2011, 35, pp. 1376-1387; Robertsen et al., 2003, supra). In Berg et al., Dev. Comp. Immunol., 2009, 33, pp. 638 - 645, the effect of an antiserum against Atlantic salmon IFNal was used to study its production in cells and neutralization of antiviral activity. IFNal had potent antiviral activity against infectious pancreatic necrosis virus (IPNV). Moreover, it was found that the antiviral activity in the supernatant of poly I:C treated TO cells was reduced by 95 - 98% by addition of antiserum against IFNa1, and concluded that most of the antiviral activity induced by poly I:C was due to IFNal and/or IFNa2. In another work, Atlantic salmon IFNal showed, however, no antiviral activity against infectious salmon anemia virus (Kileng O, Brundtland MI, Robertsen B. Infectious salmon anemia virus is a powerful inducer of key genes of the type I interferon system of Atlantic salmon, but is not inhibited by interferon. Fish Shellfish Immunol 2007;23: 378-389).

In a comparative study, Atlantic salmon IFNc showed similar antiviral activity as IFNal while IFNb showed significantly lower activity and IFNd showed no antiviral activity (Svingerud et al., J. Immunol. 2012, 189, pp 5912-5923). A rainbow trout IFNb homolog possessed little if any antiviral activity (Zou et al., 2007, *supra*). In zebrafish, antiviral activity of the IFNc homolog IFNφ2 has been demonstrated, while the IFNd homolog, zebrafish IFNφ4, showed little or no antiviral activity (Aggad et al., J. Immunol., 2009, 183, pp. 3924-393, Lopez-Munoz et al., J. Immunol., 2009, 182, pp. 3440 - 3449). This illustrates that even if a protein is identified as an IFN protein by sequence analysis, it may not possess strong antiviral activity. It is thus impossible to predict whether an interferon may be useful as a therapeutic agent in combating virus infections in farmed fish just on the basis of sequence.

In addition to antiviral activity, IFN-α and IFN-β have been shown to stimulate adaptive immune responses in mammals, acting as adjuvants. This means that mammalian type I IFNs stimulate antibody production against a protein antigen or stimulate CD8 cytotoxic T-cell responses against virus protein antigens. Mammalian type I IFNs thus showed strong adjuvant activity when administrated with influenza virus antigens either delivered as genes or recombinant proteins, and they showed strong adjuvant activity when administrated as recombinant protein with chicken gamma globulin as antigen. See James, C.M., et al., 2007, "Differential activities of alpha/beta IFN subtypes against influenza virus in vivo and enhancement of specific immune responses in DNA vaccinated mice expressing haemagglutinin and nucleoprotein.", Vaccine 25:1856-67, Proietti, E. et al., 2002" Type I IFN as a natural adjuvant for a protective immune response: lessons from the influenza vaccine model.", J Immunol 169:375-83, and Le Bon et al. , 2001, "Type I interferons potently enhance humoral immunity and can promote isotype switching by stimulating dendritic cells in vivo.", Immunity., 14(4):461-70. PubMed PMID: 11336691.

Various mechanisms seem to be involved in adjuvant activity of mammalian type I IFNs including stimulation of maturation of antigen presenting dendritic cells (Tough, D. F. 2004. Type I interferon as a link between innate and adaptive immunity through dendritic cell stimulation. Leuk Lymphoma 45:257-64) and direct stimulation of B- and T-cells (Le Bon A, Thompson C, Kamphuis E, Durand V, Rossmann C, Kalinke U, Tough DF. Cutting edge: enhancement of antibody responses through direct stimulation of B and T cells by type I IFN. J Immunol. 2006 Feb 15;176(4):2074-78). The adjuvant activity and antiviral activities of type I IFNs are thus distinct functions.

Secombes in Fish and Shellfish Immunology (2008), 25, pp. 409-416 discuss various immune genes in fish in term of their relevance to vaccine design and development. However, amongst the fish cytokines mentioned in respect of discussing possible adjuvant in fish vaccine, interferons are not mentioned.

Anderson D. P., 1992, "Immunostimulants, adjuvants, and vaccine carriers in fish: Applications to aquaculture", Annual Review of Fish Diseases, 2:281-307 describes that fish interferons are immunostimulants in fish. It is discussed that immunostimulants may be administered by themselves or with a vaccine to activate non-specific defence mechanisms.

The mammalian studies have led to the question if fish type I IFNs may also have vaccine adjuvant effects. At present, no knowledge is available making it possible to conclude that there is a direct correlation between the role and characteristics of the various types of mammalian IFNs and fish IFNs. In addition, based on the present mammalian studies, and the present thrifty and divergent knowledge of the role and characteristics of the various fish IFNs, it is not possible to draw any conclusion in respect of whether the fish IFNs, and in that case which one, or the genes encoding such fish IFNs, may have any applicability in therapy in fish farming. Without any experimental proves, one cannot predict whether any, all or some fish type I IFNs may have any vaccine adjuvant effects.

The present inventors have now shown that the administration of a fish interferon coding sequence results in an increased protection against viral infection in fish when used as an adjuvant together with a DNA vaccine against a pathogenic microorganism in fish. Furthermore, it has been found that a fish interferon coding sequence results in enhanced antibody response when used as an adjuvant together with a DNA vaccine against a protein from a pathogenic microorganism in fish.

### Summary of the invention

The present invention relates to an interferon a (IFNal) coding sequence, an (IFNa2) coding sequence, an interferon b (IFNb) coding sequence and an interferon c (IFNc) coding sequences for use as vaccine adjuvants in vaccination of farmed fish.

According to one embodiment of the invention, an interferon coding sequence is provided, wherein the sequences is selected from the group consisting of SEQ ID No. 1, SEQ ID No. 3, and SEQ ID No. 4 for the use as a vaccine adjuvant in combination with a DNA vaccine plasmid in salmon.

The interferon coding sequence for the use as a vaccine adjuvant disclosed herein is selected from the group consisting of SEQ ID No. 3 encoding IFNc or a fragment thereof, and fragments and variants of SEQ ID No. 3 having at least 70% sequence identity with the sequenceSEQ ID No. 3.

According to another embodiment of the invention, an interferon coding sequence for the use as a vaccine adjuvant is provided, wherein the sequences is SEQ ID No. 3 encoding IFNc or a fragment thereof, and fragments and variants of SEQ ID No. 3 having at least 70% sequence identity with the sequences SEQ ID No. 3.

According to one embodiment of the invention, said sequences are provided for use as a DNA vaccine adjuvant in combination with a DNA vaccine plasmid in salmon.

According to another embodiment of the invention, said sequences are provided for use as a vaccine adjuvant in order to enhance the antibody response or the cytotoxic T cell response against a fish pathogen, such as e.g. a virus, a bacterium or a parasite.

According to another embodiment of the invention, said sequences are provided for use as a vaccine adjuvant in combination with a DNA vaccine plasmid in salmon, wherein the virus is selected from the group consisting of virus selected from the group infectious pancreatic necrosis virus (IPNV), salmonid alpha virus (SAV), viral hemorrhagic septicemia virus (VHSV), Infectious haematopoietic necrosis virus (IHNV), infectious salmon anemia virus (ISAV), virus causing heart and skeletal muscle inflammation in salmon (recently identified as piscine reovirus (PRV) or Atlantic salmon reovirus), and piscine myocarditis virus (PMCV), which causes cardiomyopathy syndrome in salmon.

According to another embodiment of the invention, said sequences are provided for use as a vaccine adjuvant wherein the virus is ISAV.

According to another aspect of the present invention, a plasmid is provided comprising a polynucleotide sequence encoding IFNal (SEQ ID No. 1), , IFNb (SEQ ID NO. 3) or IFNc (SEQ ID No. 4), for use as vaccine adjuvant in combination with a DNA vaccine plasmid in salmon, wherein said polynucleotide sequence is operably linked to a promoter sequence. Said promoter sequence is according to another embodiment selected from the group consisting of cytomegalovirus (CMV) promoter, Rous sarcoma virus (RSV) promoter, simian virus 40 (SV-40) promoter, simplex herpes virus (HSV), muscular β-actin promoter, elongation factor 1 alpha promoter, desmin promoter and creatine kinase promoter. According to a preferred embodiment, said promoter is cytomegalovirus (CMV) promoter.

According to another embodiment of the invention, the plasmid of the present invention furthermore comprises a polynucleotide sequence encoding an antigen operably linked to a promoter sequence. Said promoter being operably linked to an antigen coding sequence may be the same or different from the promoter operably linked to the interferon coding sequence, and may e.g. be selected from the group consisting of cytomegalovirus (CMV) promoter, Rous sarcoma virus (RSV) promoter, simian virus 40 (SV-40) promoter, simplex herpes virus (HSV), muscular β-actin promoter, elongation factor 1 alpha promoter, desmin promoter and creatine kinase promoter. According to a preferred embodiment, said promoter is cytomegalovirus (CMV) promoter.

According to another aspect of this embodiment, the antigen originates from a virus, such as e.g. a virus selected from the group infectious pancreatic necrosis virus (IPNV), salmonid alpha virus (SAV), viral hemorrhagic septicemia virus (VHSV), Infectious haematopoietic necrosis virus (IHNV), infectious salmon anemia virus (ISAV), virus causing heart and skeletal muscle inflammation in salmon (recently identified as piscine reovirus (PRV) or Atlantic salmon reovirus), and piscine myocarditis virus (PMCV), which causes cardiomyopathy syndrome in salmon. According to one embodiment, said antigen originates from ISAV. According to yet an embodiment, said antigen coding sequence codes for hemagglutinin-esterase (HE) originating from ISAV.

According to another embodiment of the present invention, the plasmid furthermore comprises a sequence coding a selection marker, e.g. a selection marker being is selected from the group consisting of a kanamycin, ampicillin and neomycin resistance gene.

According to yet another aspect of the present invention, a vaccine composition is provided comprising:
(i) a DNA vaccine plasmid comprising a polynucleotide sequence encoding an antigen originating from a fish pathogen in salmon operably linked to a promoter; and
(ii) an interferon coding sequence selected from the group consisting of SEQ ID No. 1 encoding IFNa1, SEQ ID No. 3 encoding IFNb, and SEQ ID No. 4 encoding IFNc. Optionally, the composition may also comprise
(iii) one or more pharmaceutically acceptable carriers.

Said composition may according to one embodiment comprise a the DNA vaccine plasmid encoding an antigen originating from a virus selected from the group infectious pancreatic necrosis virus (IPNV), salmonid alpha virus (SAV), viral hemorrhagic septicemia virus (VHSV), Infectious haematopoietic necrosis virus (IHNV), infectious salmon anemia virus (ISAV), virus causing heart and skeletal muscle inflammation in salmon (recently identified as piscine reovirus (PRV) or Atlantic salmon reovirus), and piscine myocarditis virus (PMCV), which causes cardiomyopathy syndrome in salmon. Preferably, said antigen is ISAV hemagglutinin-esterase (HE).

According to yet another embodiment of this aspect of the invention, a composition is provided, wherein the interferon coding sequence being operably linked to a promoter (ii) is comprised in the DNA vaccine plasmid (i).

According to another embodiment of this aspect of the invention, a composition is provided, wherein the interferon coding sequence (ii) is present in a plasmid different from the DNA vaccine plasmid (i).

According to one aspect of the invention, the one or more polynucleotide sequence(s) encoding an antigen and the interferon coding sequence is operably linked to the same or different promoters selected from the group consisting of cytomegalovirus (CMV) promoter, Rous sarcoma virus (RSV) promoter, simian virus 40 (SV-40) promoter, simplex herpes virus (HSV), muscular β-actin promoter, elongation factor 1 alpha.

Finally, according to yet an aspect of the present invention, a recombinant IFNc protein (rIFNc) is provided for the use as a vaccine adjuvant in combination with an inactivated whole virus vaccine in salmon, wherein the recombinant IFNc (rIFNc) protein comprises a sequence of SEQ ID No. 8.

Also disclosed is the rIFNc is comprised in a fish vaccine formulation, e.g. a vaccine used in order to provide protection against one or more fish pathogens, such a microorganism(s) or a parasite(s).

The rIFN is comprised in a fish vaccine formulations used to provide protection against a virus, e.g. wherein the virus is selected from the group consisting of virus selected from the group infectious pancreatic necrosis virus (IPNV), salmonid alpha virus (SAV), viral hemorrhagic septicemia virus (VHSV), Infectious haematopoietic necrosis virus (IHNV), infectious salmon anemia virus (ISAV), virus causing heart and skeletal muscle inflammation in salmon (recently identified as piscine reovirus (PRV) or Atlantic salmon reovirus), and piscine myocarditis virus (PMCV), which causes cardiomyopathy syndrome in salmon.

### Figures

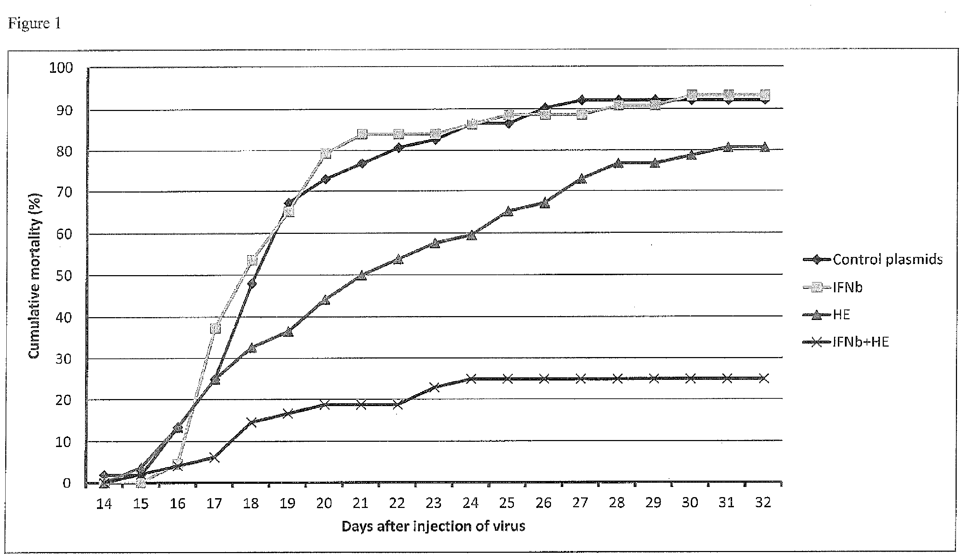

Figure 1 shows that fish injected with a DNA vaccine consisting of an expression plasmid encoding ISAV hemagglutininesterase (HE) and an expression plasmid encoding salmon IFNb resulted in lower cumulative mortality compared with fish injected with the HE encoding plasmid only, the IFNb encoding plasmid only, or the plasmid control. The fish were challenged by intraperitoneal injection of ISAV.

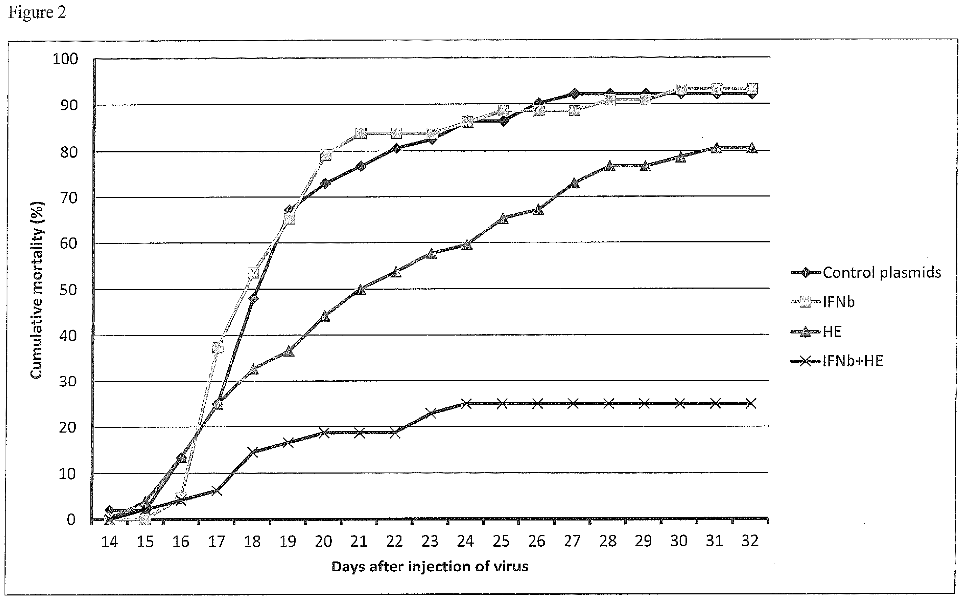

Figure 2 shows that fish injected with a DNA vaccine against ISAV HE and an expression plasmid encoding salmon IFNa or IFNc resulted in lower cumulative mortality compared with fish injected with the DNA vaccine alone, an IFNa or an IFNc encoding plasmid only, or the plasmid controls. The fish were challenged by intraperitoneal injection of ISAV.

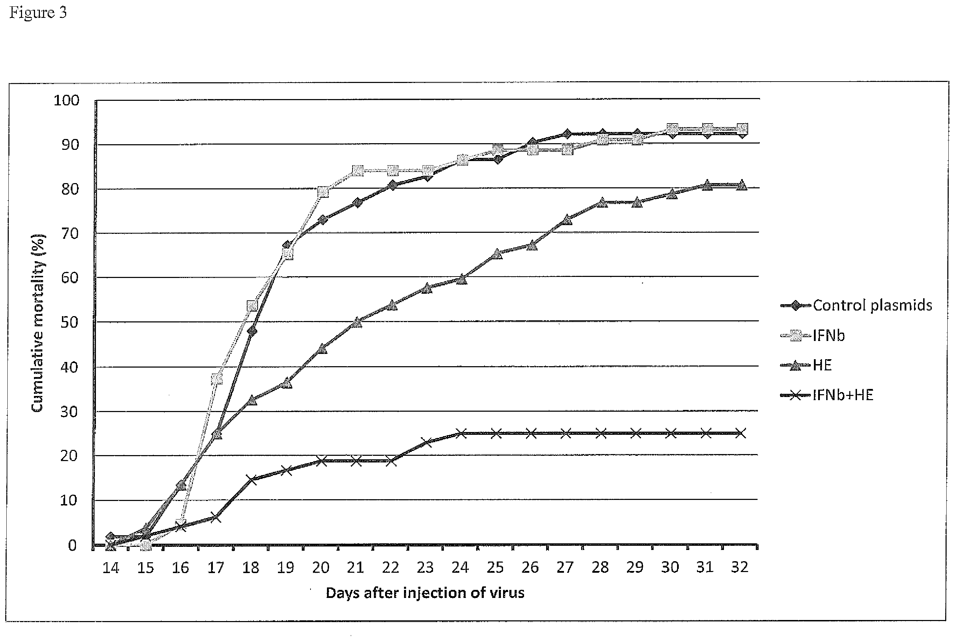

Figure 3 shows that fish injected with a DNA vaccine against ISAV HE and an expression plasmid encoding salmon IFNa, IFNb or IFNc resulted in lower cumulative mortality compared with fish injected with the DNA vaccine alone, an IFNa, IFNb or an IFNc encoding plasmid only, or the plasmid controls. The fish were challenged by cohabitation challenge with ISAV infected fish.

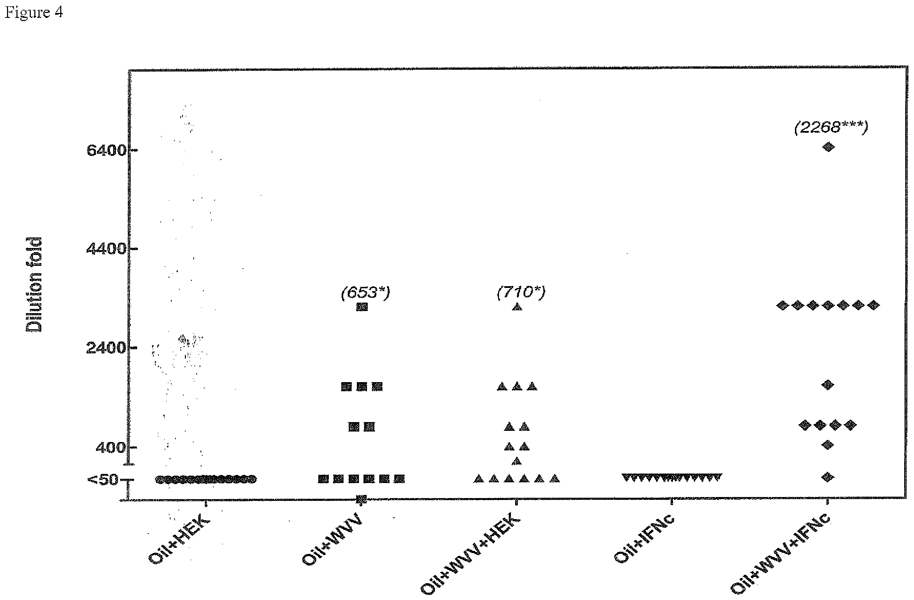

Figure 4 shows the HE specific antibody response of Atlantic salmon injected with differing plasmid DNA into the muscle at 10 weeks post vaccination. Any significant differences in geometric mean titre (gmt) are marked with an asterisk *, p<0.05 and ** means p<0.005, *** means p<0.001.

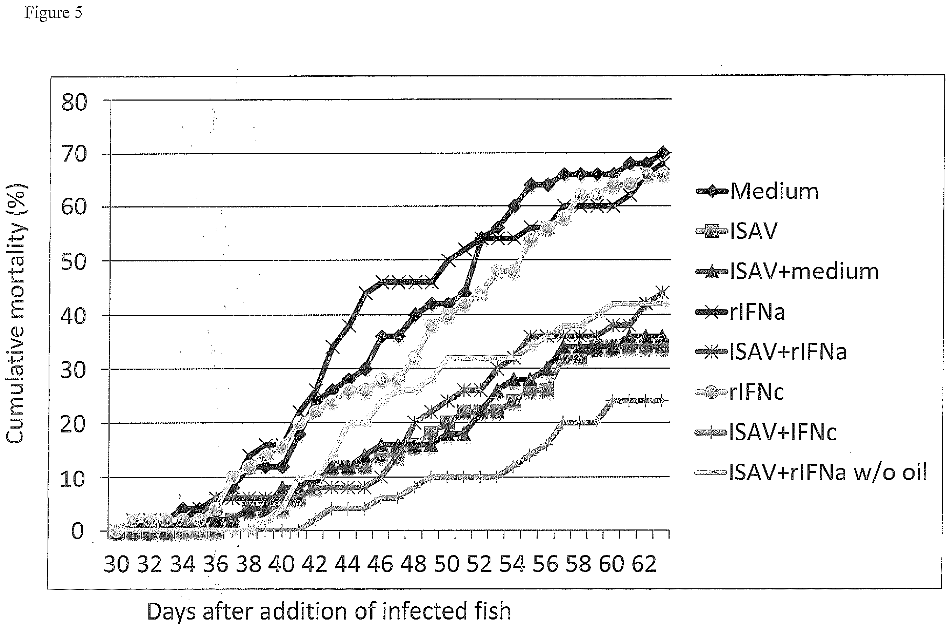

Figure 5 shows the adjuvant effect of recombinant IFNa1 and IFNc in inactivate whole virus ISAV vaccine.

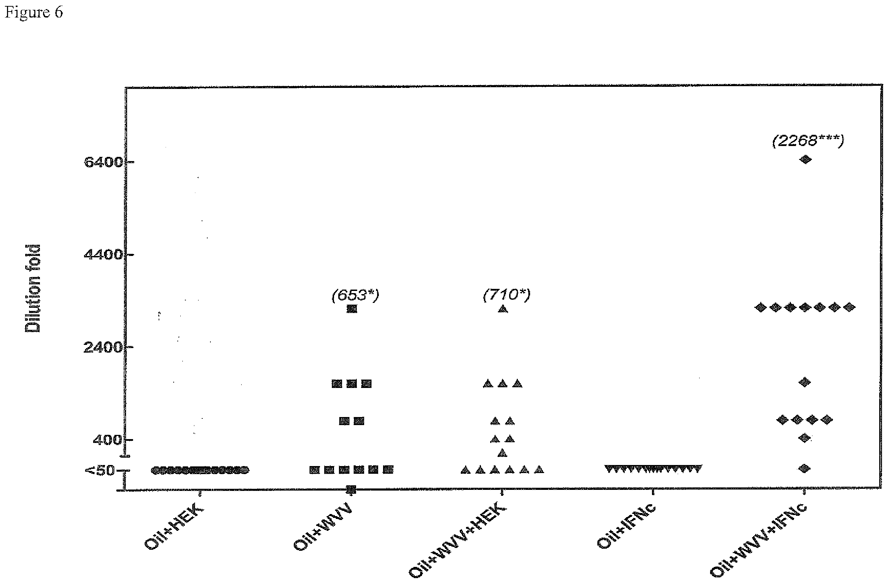

Figure 6 shows the ISAV specific antibody response of Atlantic salmon injected with WVV vaccine with and without recombinant IFNc and an oil adjuvant measured by ELISA 8 weeks after injection.

### Detailed description of the invention

In the detailed description that follows, a number of expressions and terms commonly used within recombinant technology, within immunology and vaccine technology are used. In order to provide a clear and consistent understanding of the present invention, the claims and the scope to be given such terms, specific definition are provided below.

A "coding sequence" is to be understood to mean a polynucleotide sequence which when transcribed into mRNA and/or translated into a polypeptide, i.e. when placed under the control of an appropriate control sequence such as a promoter. The starting point of the coding sequence may be determined by a translation start codon at the 5'-terminus. The end of the coding sequence may be determined by a translation stop codon at the 3'-terminus. A coding sequence may e.g. be represented by a mRNA sequence, a cDNA sequence or a genomic DNA sequence, and/or a recombinant polynucleotide sequence.

The term "interferon (IFN) coding sequence" as used is to be understood to mean a nucleic acid sequence encoding an interferon or a fragment or variant thereof. In particular, the present invention provides the use of IFNa1, IFNa2-,IFNb- and IFNc-coding sequences as adjuvant in DNA vaccines.

The gene sequences of IFNal and IFNa2 were first disclosed by Robertsen et al., 2003, *supra.* The gene sequences encoding IFNb and IFNc were first disclosed by Sun et al., 2009, *supra.* In the present examples only the IFNal sequence has been used to demonstrate adjuvant activity of IFNa since IFNa2 has 95% sequence identity with IFNa2. IFNb and IFNc are encoded by multiple genes in salmon where the IFNb sequences are 95-97% identical and the IFNc sequences are 90-99% identical. The IFNb and IFNc sequences chosen in the present examples are representatives of the most highly expressed IFNb and IFNc genes from salmon stimulated with the compound R848. The open reading frames encoding IFNa1, IFNa2, IFNb and IFNc are depicted in the sequence listing as SEQ ID NQ 1, SEQ ID No. 2, SEQ ID No. 3 and SEQ ID No. 4, respectively.

It is to be understood that the term "IFNal coding sequence", IFNa2 coding sequence", "IFNb coding sequence" and "IFNc coding sequence" means not only the sequence as specifically depicted in SEQ ID No. 1, SEQ ID No. 2, SEQ ID No 3, and SEQ ID No. 4, respectively, but also fragment and variants thereof which when expressed in a recipient animal upon administration is capable of acting as a vaccine adjuvant. The skilled person is aware of the fact that the nucleic acid sequence encoding a protein may differ to some degree without affecting the activity of the encoded protein. E.g. SEQ ID No. 1, SEQ ID No. 2, SEQ ID No. 3 or the SEQ ID No. 4 may differ by way of some nucleotide additions, deletions or alterations that have little effect, if any, on the functional activity of the resulting IFNa1, IFNa2, IFNb or IFNc encoded thereby. The skilled person is also well aware of the fact that modification of a protein coding nucleic acid sequence may be introduced, which does not alter the encoded amino acid sequence. For example, substitution of a nucleotide may result in that the triplet affected still encodes the same amino acid due to the degeneration of the genetic code. The substitution of a nucleotide may also result in the substitution of an amino acid with similar chemical characteristics, thus not affecting the structure and activity of the protein. Examples of substitutions not having effect on protein structure or activity is e.g. the substitution of aspartic acid for glutamic acid, or the substitution of lysine for arginine, or the substitution of a valine for leucine or isoleucine.

The skilled person will also acknowledge that a nucleic acid sequence may be modified in such a way resulting in that in the encoded protein, one or more amino acid(s) may be added or deleted compared with SEQ ID No. 1, SEQ ID No. 2, SEQ ID No. 3, and SEQ ID No 4, respectively, without altering the function of the resulting protein. Each of the types of modifications mentioned above is well known to the skilled person, and depends on the retention of the interferon activity of the proteins encoded by the IFNa1, IFNa2, IFNb and IFNc coding sequences in accordance with the use of said sequences. Thus, the skilled person will, based on the teaching herein and in combination of his/her common general knowledge, acknowledge that various alteration of the SEQ ID No. 1, SEQ ID No. 2, SEQ ID No 3, or SEQ ID No. 4, may be introduced which do not significantly alter the function or activity of the resulting expressed protein. The skilled person will thus acknowledge that a nucleotide sequence differing from SEQ ID No. 1, SEQ ID No. 2, SEQ ID No 3, or SEQ ID No. 4, respectively, by way of substitution(s), addition(s) or deletion(s) of nucleotides may be used according to the present invention depending on the retention of the interferon activity of the proteins encoded by the IFNa1, IFNa2, IFNb and IFNc coding sequences, respectively, in accordance with the use of said sequences.

Thus, fragments or variants of the SEQ ID No. 1, SEQ ID No. 2, SEQ ID No. 3 and SEQ ID No. 4 is to be understood to mean having at least 70% sequence identity with the said SEQ ID No. 1, SEQ ID No. 2, SEQ ID NO. 3, and SEQ ID No. 4, respectively. The term "70% sequence identity" is to be understood to refer to the percentage of nucleotides that two or more sequences or fragments thereof contains that are the same. A specified percentage of nucleotides can be referred as e.g. having 70% sequence identity, 75% sequence identity, 80% sequence identity, 85% sequence identity, 90% sequence identity, 95% sequence identity, 99% sequence identity or more over a specified region when compared aligned for maximum correspondence. The skilled person in the art is well known with various commonly used means for comparing the identity between nucleic acid sequences. Reference may e.g. be made to suitable computer programs commonly used in order to determine the % identity of two or more nucleic acid sequences, such as the Basic Local Alignment Search Tool (BLAST) (Altshul et al., J of Molecul. Biol., 1990, 215, pp. 3389-3402).

The use of the IFNa1, IFNa2, IFNb and IFNc encoding sequences according to the present invention is effectuated by inserting the said sequences in recombinant constructs resulting in the expression of said encoding sequences in the recipient fish. Said constructs comprises control sequences enabling the expression of the IFNa1, IFNa2, IFNb and IFNc encoding sequences, and may be control sequences such as promoters and enhancers.

The term "promoter" is to be understood to represent a nucleotide sequence that is comprised of a sequence recognised by RNA polymerases, which upon binding to the DNA template provides for the production of mRNA of the adjacent structural gene. A "promoter" is thus a control sequence which is necessary to effect the expression of a coding sequence to which they are ligated. A non-limiting list of promoters that may be used according to the present invention is cytomegalovirus (CMV) promoter, Rous sarcoma virus (RSV) promoter, simian virus 40 (SV-40) promoter, muscular β-actin promoter, elongation factor 1 alpha promoter. According to one embodiment of the invention, the CMV promoter is operably linked to the IFNc coding sequence in an appropriate expression plasmid.

The expression "operably linked" refers to a juxtaposition wherein the components so described are in a relationship permitting them to function in their intended manner. For example, a promoter sequence "operably linked" to a coding sequence is ligated in such a way that expression of the coding sequence is achieved under conditions suitable for the promoter sequence.

The interferon coding sequence may be introduced into the fish in any suitable form enabling expression of said coding sequence in the recipient fish. For example, the interferon coding sequence may be introduced in a linearized or circular plasmid which upon injection into the recipient fish is replicated and wherein the interferon coding sequence is expressed. Preferably, the interferon coding sequence is operably linked to a promoter and contained in a plasmid, such as e.g. an expression plasmid. Suitable expression vectors may be produced using standard recombinant techniques, cf. Sambrook and Russel, Molecular Cloning; A Laboratory Manual or DNA cloning, vol I, II and III Cold Spring Harbor Laboratory Press, 2001, pp.2344. Suitable expression plasmids adapted for expression of recombinant nucleic acid sequences in fish cells may be used to express IFNa1, IFNa2, IFNb and IFNc coding sequences according to the present invention. Several plasmids that may be useful are known in the prior art. A non-limiting list of plasmids that may be used according to the present invention is pVAX1, pcDNA3.1, pcDNA3.3-TOPO (Invitrogen), pQE-TriSystem-7 vector (Qiagen), pcDNA™ 3.2/V5-DEST vector (Invitrogen). According to one embodiment of the present invention, the IFNal sequence is inserted in the pcDNA3.1 vector and the IFNa1, IFNa2, IFNb and IFNc coding sequences are each inserted in a pcDNA3.3-TOPO vector.

A plasmid comprising the IFNa1, IFNa2, IFNb or IFNc coding sequence according to the present invention may preferably furthermore comprise marker sequence facilitating the propagation of the plasmid in a suitable host cell. The marker will ensure stable inheritance of plasmids during propagation in host cells. Most markers useful for the selection and propagation of expression plasmids rely on resistance to antibiotics. The marker may e.g. be an ampicillin or kanamycin resistance gene for selection and propagation of the plasmid according to the present invention in E. coli cells. The marker may furthermore e.g. be a neomycin resistance gene for selection of stable cell lines expressing IFNa1, IFNa2, IFNb or IFNc with Geneticin®.

Also, non-antibiotic based markers may be used. For example, a non-antibiotic-based marker useful for propagating an expression plasmid in E. coli is based on the displacement of repressor molecules from the chromosome to the plasmid, allowing expression of an essential gene (Cranenburgh et al., "Escherichia coli strains that allow antibiotic-free plasmid selection and maintenance by repressor titrations", 2001, Nucleic Acid Res., 29: E26). Another non-antibiotic based marker is based on a biocide triclosan as selective agent and an endogenous growth essential target gene, fabI, as a marker for propagation of plasmid in E. coli (El-Attar et al., "A pest virus DNA vaccine based on a non-antibiotic resistance Escheriachia Coli Essential gene marker", 2012, Vaccine, 30(9), pp 1702-1709). Another non-antibiotic based selection marker than may be used according to the present invention is an araD gene encoding L-ribulose-5-pospate-4-epimerase, cf. US patent No. 7,521,182. Yet another non-antibiotic selection system is based on complementation of host auxotrophy in the NAD de novo synthesis pathway (Dong et al., Novel antibiotic-free plasmid selection system based on complementation of host auxotrophy in the NAD de novo synthesis pathway, Appl. Environ. Microbiol., 2010, 76(7), pp.2295-2303).

Expression plasmids according to the present invention may be propagated and expressed in any suitable host cell known to the skilled person. For example, the expression plasmid according to the present invention may be propagated in a prokaryotic host cell or eukaryotic host cells. According to one embodiment of the present invention, the expression plasmid is propagated in a prokaryotic host cell, such as *E. coli.*

The present inventors have furthermore surprisingly found that a recombinant IFNc protein may be used as a vaccine adjuvant. A recombinant IFNc protein may be prepared by conventional method for the preparation and expression of expression plasmids. For example, an IFNc coding sequence or a variant or fragment thereof may be ligated into a suitable expression plasmid, and the expression plasmid may furthermore be introduced into a suitable host cell, from where the resulting recombinant protein is isolated and optionally further purified. Various expression systems and host cells may be used in order to prepare a recombinant IFNc protein to be used according to the present invention. The skilled person is well familiar with the various available biotechnological techniques providing for the expression of protein coding nucleic acid sequences for the preparation of recombinant proteins by heterologous expression in suitable host cells using commonly available techniques and protocols, cf. e.g. "Recombinant Gene Expression Protocols, in Methods in Molecular Biology, 1997, Ed. Rocky S Tuan, Human Press (ISSN 1064-3745) or Sambrook et al., Molecular Cloning: A laboratory Manual (third edition), 2001, CSHL Press, (ISBN 978-087969577-4). For example, the nucleic acid sequences according to SEQ ID No. 4 or fragments or variants thereof may be ligated into suitable expression vectors comprising all the necessary transcriptional and translational regulatory sequences specifically adapted for directing the expression of the desired protein coding nucleic acid sequence in a suitable host cell. The IFNc coding sequence or a fragment or variant thereof is thus operably linked to a promoter and optionally further sequences directing the expression of the said sequence. A non-limiting list of examples of suitable expression vector systems is e.g. pET-type of vectors, pcDNA3.1 vectors, or the pBR-type, pUC-type or pGEM-type of expression vectors. The obtained expression vector may then be introduced in a suitable host cell, which upon culturing in a suitable broth results in the production of the desired recombinant protein.

As outlined above, it should be understood that various modifications may be introduced in the IFNc coding sequences as outlined above. In respect of the production of a recombinant protein, the skilled person will be aware of the fact that modification may also be introduced, e.g. to facilitate isolation and purification, i.e. by adding a sequence coding for a peptide or protein useful for such purposes, or for the introduction of triplets specifically preferred by the host cells expression system. Also, nucleic acid sequences coding signal peptide providing for secretion of the desired recombinant protein from the host cell may be linked to the IFNc coding sequence.

A large number of host cells specifically adapted for the production of recombinant proteins are available and may be used to prepare a recombinant IFNc protein, including prokaryotic and eukaryotic host cells. A non-limiting list of suitable prokaryote host cells is e.g. *Escherichia coli*, *Bacillus substilis*, *Lactobacillus sp*, *Caulobacter cresentus*, *Yersinia ruckeri*, *or Vibrio anguillarum*. Non-limiting examples of suitable eukaryote host cells is e.g. yeast such as *Saccharomyces cerevisiae and Pichia pastoris* (PichiaPink™ Yeast Expression System, Invitrogen) *or mammalian cell lines such as Chinese hamster ovary cells and human embryo kidney 293 cells (HEK293).* Also viruses may be used for the production recombinant expression of a desired protein, cf. e.g. alphavirus, adenovirus or baculovirus vector systems (Bac-to-Bac® Baculovirus Expression Systems, Invitrogen,).

According to one embodiment of the present invention, a recombinant IFNc is prepared using pcDNA3.3-TOPO vector (Invitrogen) as expression plasmid and HEK293T as host cell.

The rIFNc protein may be comprised in a vaccine as an adjuvant together with one or more live, attenuated or inactivated pathogenic organisms, such as virus, bacteria or parasites, or antigenic proteins originating from pathogenic organisms such as herein. The skilled person will be well aware of the various methods for preparing vaccine composition to be injected in farmed fish. A vaccine composition comprising a recombinant IFNc protein may thus in addition to the antigenic component, comprise one or more suitable pharmaceutically acceptable carrier(s) or other adjuvants well known to the skilled person as suitable when formulated vaccine composition within the aquaculture field.

The term "adjuvant" as used herein refers to a compound that enhances the immune response against an antigen presented to a recipient fish by vaccination. When the IFNa1, IFNa2, IFNb and IFNc coding nucleic acid sequences according to the present invention is used as an adjuvant, the recipient fish is administered with a composition comprising the IFNa1, IFNa2, IFNb or IFNc coding nucleic acid sequence and means for expressing said nucleic acid sequence in the recipient fish.

In fish farming, the infection of various pathogens constitutes a large economical problem to the fish farming industry. Today, a range of vaccines against various pathogens is available to the fish farmer, in particular useful in combating bacterial pathogens. Commercial vaccines against various viruses are also available, although the effect thereof is heavily debated. Thus, even if vaccines against several viruses are available, virus diseases still represent a threat and results in huge economical loss to the fish farming industry. A non-limiting list of viruses cause diseases that constitute a continuous problem within farming of salmonids, is e.g. infectious pancreas necrosis virus (IPNV), salmonid alpha virus (SAV) causing pancreas disease, infectious salmon anaemia virus (ISAV), viral hemorrhagic septicaemia virus (VHSV), infectious haematopoietic necrosis virus (IHNV), virus causing heart and skeletal muscle inflammation in salmon (recently identified as piscine reovirus (PRV) or Atlantic salmon reovirus), and piscine myocarditis virus (PMCV), which causes cardiomyopathy syndrome in salmon. Thus, there is a need for means that may improve the effect of viral fish vaccines. The present invention presents a solution to this problem by using an IFNa1, IFNa2, IFNb or IFNc coding sequence, or a fragment or variant thereof, as a vaccine adjuvant.

DNA vaccines directed towards viral pathogens have been presented as an attractive alternative to traditional vaccines (i.e. inactivated or attenuated strains of the pathogens of interest, or vaccines based on viral proteins or fragments thereof). There are several advantages of DNA vaccines that may be mentioned, for example, there is no risk of reversion to a pathogenic form, there is no problem with chemical impurities and there are less unwanted side effects compared to oil based vaccines.

As shown in the following examples, fish being administered a DNA vaccine consisting of a plasmid encoding an antigen originating from ISAV, e.g. the hemagglutinin esterase, which at the same time is administrated with a plasmid containing the interferon coding sequences according to the present invention, develops an improved protection against said pathogen compared with fish only being administered the ISAV DNA vaccine. Thus, the IFNa1, IFNa2, IFNb and IFNc coding sequences have been proven to provide an increased response to an antigen thus showing the said sequences surprising potential as (DNA) vaccine adjuvants.

According to one embodiment of the present invention, the IFNa1, IFNa2, IFNb and IFNc coding sequences are each used together with a DNA vaccine in order to improve the response to said vaccine in the recipient fish.

A DNA vaccine is constructed in order to be able to express nucleic acid sequences encoding antigens upon administration to the recipient animal. A DNA vaccine thus consists of e.g. naked plasmid DNA comprising a nucleic acid coding an antigen operably linked to a promoter and optionally other suitable control sequences enabling the expression of said antigen coding sequences by the cells of the recipient fish after administration thereof. A DNA vaccine may alternatively be carried in a live host microorganism being involved in the delivery of the genetic material to the recipient fish.

The IFNa1, IFNa2, IFNb and IFNc coding sequences, or a fragment or variant thereof, operably linked to a suitable promoter and optionally other control sequences, may according to the present invention be used as an adjuvant enhancing the response to various DNA fish vaccines.

Several fish DNA vaccines are known. For example, as early as in 1996, Anderson et al. reported that the injection of a DNA comprising infectious haematopoietic necrosis virus G and N genes present in an expression plasmid exerted protection against a live virus upon administration of said DNA vaccine into rainbow trout (Molecular Marine Biology and Biotechnology 1996, 5, pp. 114-122). Somewhat later, other groups reported of the use of a DNA vaccine against viral haemorrhagic septicaemia virus in rainbow trout and infectious salmon anaemia virus in Atlantic salmon, respectively (Fish Shellfish Immunol., 1998, 8 (4), pp. 271 - 286, Lorenzen and LaPatra, Rev. Sci.Tech., Off Int epiz., 2005, 1, pp. 201-213, Mikalsen et al., Vaccine, 2005, 23 (30), pp. 4895-4905). Heras et al furthermore reports of an in vitro and in vivo immune response induced by a DNA vaccine encoding the VP2 gene of the infectious pancreatic necrosis virus (IPNV), see Fish Shellfish Immunol., 2009, 27, pp. 120-129.

In EP 1 818 406 A1, a DNA vaccine for vaccination of aquatic animals are disclosed wherein a mammalian cytomegalovirus promoter and a expression enhancing sequence is used to express antigens of interest for obtaining protection against pathogenic infection of farmed fish.

WO 2007/031572 discloses antigen coding sequences originating from salmonids alpha virus and their use inter alia in DNA vaccines.

DNA vaccines have also been prepared for carp, cf. e.g. WO 2009/002376 and EP 2011 876 A1 disclosing DNA vaccines useful in the prevention of virus infection in carp.

According to one embodiment of the invention, the IFNa1, IFNa2, IFNb or IFNc encoding sequences is used as an adjuvant together with a DNA vaccine encoding an antigen originating from a fish pathogen or a fish parasite.

Based on the common general knowledge of the skilled person relating to the preparation of DNA vaccines and the teaching set out herein, the skilled person will be able to identify various DNA vaccines that may advantageously be improved if used together with the IFNa1, IFNa2, IFNb or IFNc coding sequences as adjuvants according to the present invention.

The term "antigen" or "antigen of interest" as used herein refers to a protein or a fragment thereof originating from a pathogenic organism and which upon administration to a recipient fish results in that the recipient fish develops a resistance against infection by said pathogen.

The term "antigenic component" as used herein refers to a live, attenuated or inactivated pathogenic organism, which may be included in a vaccine together with the rIFNc used according to the present invention.

According to one embodiment of the present invention, the origin of said antigen or "antigenic component" may be any fish pathogen, i.e. any viral, bacterial or parasitic pathogen resulting in an infectious disease in fish or any parasite of fish such as sea lice or amoebas causing gill diseases. An "antigen coding sequence" as used herein is meant to cover a nucleic acid sequence coding an "antigen" as defined above.

A non-limiting list of viruses which may be the origin of the antigen coding sequence according to the present invention is e.g. infectious pancreatic necrosis virus (IPNV), salmonid alpha virus (SAV), viral hemorrhagic septicemia virus (VHSV), infectious haematopoietic necrosis virus (IHNV), infectious salmon anemia virus (ISAV), virus causing heart and skeletal muscle inflammation in salmon (recently identified as piscine reovirus (PRV) or Atlantic salmon reovirus), and piscine myocarditis virus (PMCV), which causes cardiomyopathy syndrome in salmon.

A non-limiting list of bacterial pathogens, which may be the origin of the antigen coding sequence according to the present invention is e.g. *Aeromonas salmonicidae*, *Aeromonas hydrophila*, *Moritella viscosa*, *Flavobacterium columnaris*, *Piscirickettsia salmonis*, *Vibrio salmonicidae*, and *Vibrio anguillarum*.

A non-limiting list of parasites, which may be the origin of the antigen coding sequence according to the present invention is e.g. *Lepeophtheirus salmonis*, *Gyrodactilus salaris*, *Neoparamoeba perurans*, *Tetracapsuloides bryosalmonae*, *Ichthyophthirius multifiliis.*

The skilled person will acknowledge that antigen coding sequence may be obtained isolated from samples of a virus using well known cloning techniques. Various antigen coding sequences in addition to the one mentioned above are available, e.g. salmonid alpha virus antigen coding sequences, see US2011013569, *Piscirickettsia salmonis* nucleic acid sequences, see WO2009077577, *A. hydrophila* nucleic acid sequences, see US2012297948.

The IFNa1, IFNa2, IFNb or IFNc coding sequences, or fragments or variants thereof, operably linked to a suitable promoter may be administered together with a desired DNA vaccine, i.e. being contained in the same vaccine composition. The IFNa1, IFNa2, IFNb or IFNc coding sequences, operably linked to a promoter, may be inserted in the desired DNA vaccine in a suitable insertion site. The expression of IFNa1, IFNa2, IFNb or IFNc coding sequences may furthermore be controlled by the same promoter as controlling the expression of the desired antigen, or by different promoters.

According to another embodiment, the IFNa1, IFNa2, IFNb or IFNc coding sequences, respectively, may be present in a separate expression plasmid comprised in the same pharmaceutical composition as the DNA vaccine to be administered together to the fish recipient. The expression of the antigen and the IFNa1, IFNa2, IFNb or IFNc coding sequences, respectively, may for this embodiment also be directed by the same or by different promoters.

I.e., according to one embodiment, a composition is provided comprising a plasmid comprising an IFNa1, or IFNa2 coding sequence and a DNA vaccine plasmid comprising an antigen coding sequence. According to another embodiment, a composition is provided comprising a plasmid comprising an IFNb coding and a DNA vaccine plasmid comprising an antigen coding sequence. According to yet another embodiment, a composition is provided comprising an plasmid comprising an IFNc coding and a DNA vaccine plasmid comprising an antigen coding sequence.

Furthermore, according to yet another embodiment, a composition is provided comprising a plasmid comprising an IFNa1 or IFNa2 coding sequence and an antigen coding sequence, and wherein the expression of said sequences is under control of the same or different promoters. Yet according to another embodiment, a composition is provided comprising a plasmid comprising an IFNb coding sequence and an antigen coding sequence, and wherein the expression of said sequences is under control of the same or different promoters. Yet according to another embodiment, a composition is provided comprising a plasmid comprising an IFNc coding sequence and an antigen coding sequence, and wherein the expression of said sequences is under control of the same or different promoters.

The IFNa1, IFNa2, IFNb or IFNc coding sequences may according to the present invention also be linked directly (fused) to an antigen coding sequence.

According to yet another aspect, a composition is provided comprising a plasmid encoding an IFNa1, or IFNa2-sequence-antigen fusion protein, or an IFNb-antigen fusion protein, or an IFNc-antigen fusion protein, respectively.

The interferon coding sequence and a desired vaccine may according to another embodiment of the invention be administered in two separate compositions.

The IFNa1, IFNa2, IFNb and IFNc coding sequences, i.e. when comprised by suitable expression plasmid according to the present invention, may be administered to the recipient fish by any suitable method enabling the transfer of the plasmid/interferon coding sequence resulting in the expression of said sequence in the recipient fish. For example, the plasmid may be administered to the recipient fish by injection into the muscle tissue (in particular into the epaxial muscle) or injection into the peritoneum or by intradermal injection by particle mediated delivery using gene gun technology. The plasmid may also be delivered through the feed or by immersing the fish in a plasmid dissolved in fresh water or seawater. Several means for administering DNA vaccines to fish recipients are known in the prior art, see e.g. Heppell and Davis, "DNA Vaccines: methods and protocols" in Methods of Molecular Medicine, 2000, 29 (ISBN 978-0-89603-508-5). The same methods are useful for administering the IFNb and IFNc coding sequences used according to the present invention.

The plasmid(s) of the present invention may be administered in a pharmaceutical composition comprising the interferon coding sequence, e.g. contained in a suitable expression plasmid, and optionally a DNA vaccine, and optionally one or more pharmaceutically acceptable carriers. Suitable pharmaceutically acceptable carriers useful as excipients for formulations comprising nucleotide sequences as the active ingredient, such as plasmids or DNA vaccines, are well known to the skilled person in the art (See for example Kutzler and Weiner, Nature Reviews Genetics, 2008, 9, pp 776-788). Suitable carriers may e.g. include aqueous or non-aqueous carriers. An aqueous carrier suitable for the administration of polynucleotide sequences to a fish recipient is e.g. water, sodium chloride solutions, phosphate buffered saline (PBS), Ringer's dextrose and sodium chloride, lactated Ringer's, etc. A composition according to the present invention may furthermore comprise e.g. preservatives, such as e.g. formalin, and/or antimicrobial agents, such as e.g. polymyxins B or amphotericin B, and/or antioxidants, chelating agents.

The composition according to the present invention may furthermore comprise non-aqueous carriers well known to the skilled person. Such carriers may e.g. be included in order to facilitate the administration and uptake of the interferon coding sequence in the recipient fish and to further enhance the immune response. Such well known carriers include but are not limited to e.g. liposomes, biodegradable microparticles, aluminium hydroxide, polymers (e.g. polylactic-coglycolides or chitosan), polyethyleneimine, amine-functionalized polymethacrylates, polyvinylpyrrolidone, surface active compounds (e.g. lysolecithin, polyanion, peptides, oil or hydrocarbon emulsions, keyhole limpet hemocyanins, dinitrophenol, calcium phosphate, etc).

Upon administration of the interferon coding sequence to the recipient fish according to the present invention, said sequence will be expressed in a sufficient amount resulting in that an increased antibody response and/or cytotoxic T-cell response against the pathogen of interest is provided. Based on the teaching herein and the common general knowledge, the skilled person will acknowledge that an effective dose of the interferon coding sequence may vary dependent on the stage, state and type of fish to be vaccinated, and thus be able to adjust the dose accordingly. An effective amount of an expression plasmid according to the present to be administered may e.g. be in the range of 0.1 - 50 µg in 1-100 µl PBS.

The following non-limiting examples will further illustrate the present invention.

### Examples

### Example 1. Protection of Atlantic salmon against ISAV infection by intramuscular (i.m.) injection of an IFN expression plasmid together with a hemagglutininesterase expression plasmid. Infection by intraperitoneal injection of ISAV.

### Expression plasmids

The expression plasmid of IFNa1 is described in Robertsen et al. (2003) and consists of the open reading frame (ORF) of IFNa1 inserted downstream of the CMV promoter of the eukaryotic expression vector pCR3.1 (Invitrogen). IFNb (Gene bank accession number: JX524152) and IFNc gene (Gene bank accession number: JX524153) were cloned from a head kidney cDNA library of Atlantic salmon made from RNA harvested 12 hours after intraperitoneal (i.p.) injection of 50 g fish with 0.5 mg/ml R848. The primers used for cloning were:
IFNb forward: AACATGGCTGTATTGAAATGGTTGAG (SEQ ID No. 9)
IFNb reverse: TCACAGCTTGACTCTGCTGTCAATG (SEQ ID No. 10)
IFNc forward: AGAATGGCACTTCAGACTATCACTTGG (SEQ ID No. 11)
IFNc reverse: TCATGTTCTGTTGGCCCACAGAAGG (SEQ ID No. 12)

Amplification of ORFs by PCR was done as described by Robertsen et al (2003), and the products (SEQ ID No. 1 and SEQ ID No. 3, respectively) were inserted downstream of the CMV promoter of the eukaryotic expression vector pcDNA3.3-TOPO (Invitrogen) as described in the manual from Invitrogen.

The ISAV hemagglutininesterase DNA vaccine is described by Mikalsen et al. (2005) and consists of ISAV hemagglutininesterase fused to the N-terminus of EGFP in the eukaryotic expression plasmid pEGFP-N1 (Clontech Laboratories, Paolo Alto, CA, USA). The pEGFP-N1 plasmid was used as negative control.

### Immunization of Atlantic salmon

Experiments were carried out with healthy Atlantic salmon presmolts of the strain Aquagen standard (Aquagen, Norway) kept at Tromsø Aquaculture Research Station (Tromsø, Norway) in 300 1 tanks containing fresh water at 10°C and were fed commercial dry food. Prior to injection of plasmids, the fish were anesthetized with 0.005% benzocaine (ACD Pharmaceuticals, Norway) and tagged by tattooing (2% alcian blue, Panjet inoculator) . All animal handling was in accordance with the Norwegian "Regulation on Animal Experimentation" and the *in vivo* experiment was submitted to and approved by the Norwegian Animal Research Authority (NARA) before initiation.

Groups of 50 salmon presmolts (40 g) were injected i.m. approximately 1 cm below the dorsal fin with two expression plasmids (15 µg of each) dissolved in 50 µl PBS as described below, HE = plasmid expressing the hemagglutininesterase-EGFP, IFNa1= plasmid expressing salmon IFNa1, IFNb=plasmid expressing salmon IFNb, IFNc=plasmid expressing salmon IFNc. Group 1 is the control group, which was injected with the two control plasmids pcDNA3.3 and pEGFP N1. The fish were kept in fresh water at 10°C for 7 weeks before challenge with ISAV.

### Tank 1. IFNb as adjuvant in ISAV HE DNA vaccine experiment.

Group 1: pcDNA3.3 (control plasmid 1) + pEGFP N1 (control plasmid 2).
Group 2: IFNb + pEGFP N1
Group 3: HE-EGFP + pcDNA3.3
Group 4: HE-EGFP + IFNb

### Tank 2. IFNa and IFNc as adjuvants in ISAV HE DNA vaccine experiment.

Group 1: pcDNA3.3 (control plasmid 1) + pEGFP N1 (control plasmid 2).
Group 2: IFNa1 + control plasmid 2
Group 3: IFNa1 + HE
Group 4: IFNc + control plasmid 2
Group 5: IFNc + HE
Group 6: HE + control plasmid 1.

### Challenge with ISAV and recording of mortality development.

Seven weeks after injection of plasmids, each fish was injected i.p. with 10⁵ virus of the ISAV Glesvaer/2/90 isolate obtained from the Norwegian Veterinary Institute in Oslo. The ISAV isolate was propagated and titrated in ASK-cells (Atlantic Salmon Kidney cells) obtained from ATCC (lgcstandards-atcc.org) grown on L15 Glutamax medium containing 1x MEM Non-Essential Amino Acid Solution (Invitrogen), 100 U/ml penicillin, 100 µg/ml streptomycin and 10 % FBS. Cumulative mortality was recorded as shown in Figure 1 and Figure 2. Mortality started at day 14 and the experiment was stopped at day 32 when mortality in the control group had reached 92%. The final mortality and relative percent survival (RPS), calculated as [1 - % mortality in test group/% mortality in control group]x100, is shown in Table 1. The results from Tank 1 show that the mortality in fish injected with an IFNb encoding expression plasmid when challenge with ISAV was similar as for fish injected with the control plasmid and reached approximately 92% mortality (Fig. 1). The fish injected with the HE-expressing plasmid showed lower development of mortality, and finally reached 81 % mortality and an RPS of 12.4%. In contrast, the fish injected with the HE-plasmid together with the IFNb-plasmid developed much lower mortality and reached a final mortality of 25% and an RPS of 72.8%. In Tank 2, fish injected with IFNa1 encoding expression plasmid showed only slightly less mortality (88 %) compared to fish as fish injected with control plasmids (96%) (Fig.2). Fish injected with the HE expressing plasmid had a final mortality of 74.9% and an RPS of 22%. In contrast, the fish injected with the HE-plasmid together with the IFNal-plasmid developed much lower mortality and reached a final mortality of 35% and an RPS of 64%. Fish injected with the IFNc encoding expression plasmid showed significantly reduced cumulative mortality (50%) compared with the control plasmid and fish injected with IFNal encoding expression plasmid. However, fish injected with the HE-plasmid together with the IFNc-plasmid developed even lower mortality and reached a final mortality of 26 % and an RPS of 72.9%.

**Table 1**

| Summary of cumulative mortality and relative percent survival (RPS) of DNA vaccinated Atlantic salmon in Tank 1. | | |
|---|---|---|
| Injected plasmids | Cumulative mortality (%) | RPS |
| pcDNA3.3+ EGFP N1 | 92 | - |
| IFNb + EGFP N1 | 93.2 | 0 |
| pcDNA3.3+HE-EGFP | 80.6 | 12.4 |
| IFNb+HE-EGFP | 25 | 72.8 |

**Table 2**

| Summary of cumulative mortality and relative percent survival (RPS) of DNA vaccinated Atlantic salmon in Tank 2. | | |
|---|---|---|
| Injected plasmids | Cumulative mortality (%) | RPS |
| pcDNA3.3+ EGFP N1 | 96 | - |
| IFNa1 + EGFP N1 | 88.2 | 8.1 |
| pcDNA3.3+HE-EGFP | 74.9 | 22.0 |
| IFNa1 +HE-EGFP | 34.6 | 64.0 |
| IFNc + EGFP N1 | 49.9 | 48.0 |
| IFNc + HE-EGFP | 26 | 72.9 |

The results of the above experiments are also shown in figure 1 and 2.

Taken together these results demonstrates that all three IFN- expressing plasmids, IFNa1, IFNb and IFNc, had a strong ability to enhance the protective immune response against ISAV infection obtained by DNA vaccination of salmon with the plasmid expressing ISAV hemagglutininesterase.

### Example 2

### Protection of Atlantic salmon against ISAV infection by intramuscular (i.m.) injection of an IFNa or IFNc expressing plasmid together with a hemagglutininesterase expression plasmid. Infection by cohabitation with salmon injected with ISAV.

### Immunization of Atlantic salmon

Groups of 50 salmon presmolts (40 g) were injected i.m. approximately 1 cm below the dorsal fin with plasmid(s) dissolved in 50 µl PBS as described in Table 3:

**Table 3. Summary of plasmids injected into each of the fish groups.**

| | **Vaccine** |
|---|---|
| Group 1 | 15 µg pcDNA3.3+ 15 µg pEGFP-N1 |
| Group 2 | 15 µg IFNa1 |
| Group 3 | 15 µg IFNa1+ 15 µg pEGFP-HE |
| Group 4 | 15 µg IFNc |
| Group 5 | 15 µg IFNc + 15 µg pEGFP-HE |
| Group 6 | 15 µg pEGFP-HE |

HE = plasmid expressing the hemagglutininesterase-EGFP, IFNa1= plasmid expressing salmon IFNa1, IFNb=plasmid expressing salmon IFNb, IFNc=plasmid expressing salmon IFNc. pcDNA3.3 is the control plasmid for the IFN expressing plasmids and pEGFP-N1 is the control plasmid for the HE expressing plasmid.

The fish were kept in one 300 1 tank with running fresh water at 10°C for 7 weeks before challenge with ISAV by addition of 60 fish injected i.p. with 10⁵ virus of the ISAV Glesvaer/2/90. Cumulative mortality was recorded as shown in Figure 3. Mortality started at day 36 and the experiment was stopped at day 62 when mortality in the control group had reached 70%. The final mortality and relative percent survival (RPS) is shown in Table 4 and figure 3.

**Table 4. Summary of cumulative mortality and relative percent survival (RPS) of DNA vaccinated Atlantic salmon after cohabitant challenge with ISAV.**

| **Groups of plasmid injected fish** | **Cumulative mortality** | **RPS** |
|---|---|---|
| pcDNA3.3+pEGFP-N1 | 70% | - |
| IFNa1 | 66% | 5.7% |
| IFNal+ pEGFP-HE | 6% | 91.4% |
| IFNc | 18% | 74.5% |
| IFNc + pEGFP-HE | 10% | 85.7 % |
| pEGFP-HE | 40% | 42.9% |

The results from this experiment show that fish injected with the IFNa1-expression plasmid showed only slightly less mortality (66 %) compared to fish as fish injected with control plasmids (70%) (Fig.2). Fish injected with the HE-expression plasmid had a final mortality of 40% and an RPS of 42.9%. In contrast, the fish injected with the HE-plasmid together with the IFNa1-plasmid developed much lower mortality and reached a final mortality of 6% and an RPS of 91.4%. Fish injected with the IFNc-expression plasmid reached a final mortality of 6% and an RPS of 91.4% and thus showed significantly reduced cumulative mortality compared with the control plasmid and fish injected with IFNa1-expression plasmid. However, fish injected with the HE-plasmid together with the IFNc-plasmid developed even lower mortality and reached a final mortality of 10 % and an RPS of 85.7 %.

### Example 3

### Humoral immune response against ISAV of Atlantic slamon injected i.m. with an IFN expression plasmid together with a hemagglutininesterase expression plasmid.

### Immunization of Atlantic salmon

Groups of 50 Atlantic salmon presmolts (40 g) were injected i.m. approximately 1 cm below the dorsal fin with two expression plasmids (15 µg of each) dissolved in 50 µl PBS as described in Table 5.

**Table 5.**

| | **Vaccine** |
|---|---|
| Group 1 | pcDNA3.3+pEGFP-N1 |
| Group 2 | IFNa+pEGFP-N1 |
| Group 3 | 3. IFNa+ pEGFP-HE |
| Group 4 | 4.IFNb+pEGFP-N1 |
| Group 5 | 5.IFNb+pEGFP-HE |
| Group 6 | 6.pcDNA3.3+pEGFP-HE |
| Group 7 | 7.IFNc+ pcDNA3.3 |
| Group 8 | 8.pcDNA3.3+pEGFP-HE |

### Measurement of antibody titers against ISAV

Serum was sampled from 8 fish at 10 weeks after imunization. ELISA was used to determine ISAV-specific antibodies in salmon sera using ISAV as antigen. ISAV4 was propagated in TO cells grown in L-15 medium in 75 cm³ tissue culture flasks virus. When CPE was apparent, virus was isolated from the medium by ultracentrifugation. Microtitre plates (Immuno-Plate Maxisorp, Nunc) were coated overnight at 4°C with ISAV corresponding to 200 ng protein in 100 µl 0.1 M sodium carbonate buffer (pH 9.6) in each well. The plates were then washed and blocked with 5% skim milk in TBST buffer (50mM Tris-HCl, 150 mM NaCl, 0.1% Tween 20, pH 7.6). Fish sera were two-fold diluted in TBST buffer from 1:50 to 1:12800 fold dilution and 100 µl of each dilution was added to each antigen-coated well and incubated over night at 4°C. Monoclonal mouse anti-salmon Ig and subsequently peroxidase conjugated polyclonal goat anti-mouse Ig were added in dilutions of 1:300 and 1:2000 in TBST buffer and incubated at room temperature for 60 min, respectively. TMB substrate was added and reacted for 10 min after which the reactions were stopped with 3M H₂SO₄ stop solution and optical density (OD₄₅₀nm) measured. Serum from an ISAV-infected fish was used as the positive control at two fold dilutions from 6400 to 204800 and the standard deviation was calculated. All sera were tested in duplicates. The antibody titre was determined as the reciprocal mean value of the highest dilution of each tested sera, which was 2 SD above the mean value of the same serum dilution from fish injected with control plasmids. The value of serum in each fish was plotted as a column using scatter graph (GraphPad Prism Version 5.0, San Diego). If the serum value was lower than it's starting dilution of 1:50 then the titre was ascribed a value of 25 when calculating the geometric mean titre (gmt). The gmt mean of each group was printed above its column. Significant differences in gmt between different fish in each group were analysis by Kruskal-Wallis test method for all the values of all groups (GraphPad Prism Version 5.0, San Diego).

Figure 4 shows the antibody titers against ISAV in sera from the different vaccine groups.

The data show that the antibody titres of sera from fish injected with IFN-plasmids injected together with the EGFP-N1 control plasmid (IFNa+EGFP, IFNb+EGFP and IFNc+EGFP) were not significantly higher than the antibody titres of sera from fish injected with the pcDNA3.3 and the EGFP-N1 plasmid (CP1+EGFP) and ranged from 72 to 462. The antibody titer of sera from fish injected with the HE-EGFP plasmid together with pcDNA3.3 were significantly higher than titers from fish injected with the two control plasmids (p<0.05), but were not significantly different from antibody titres in fish injected with an IFN-plasmid together with the EGFP-plasmid. In contrast, sera from fish injected with an IFN-plasmid together the HE-EGFP plasmid all contained markedly higher antibody titres compared to the sera from the control fish and fish injected with the HE-EGFP plasmid together with pcDNA3.3. In fact, the mean antibody titres of fish injected with IFNa+HE, IFNb+HE or IFNc+HE plasmids were 9603, 8803 and 9600, respectively, whilst the mean antibody titre of fish injected with pcDNA3.3+ HE plasmids was only 412.

### Example 4. Use of rIFNc as adjuvant in a vaccine based on inactivated whole virus.

In this experiment recombinant IFNal was produced in *E. coli* and IFNc was produced in HEK293T cells. ISAV was inactivated in formalin. Inactivated virus and IFNs were mixed and formulated as a water-in-oil emulsion to produce the vaccine. Atlantic salmon presmolts were vaccinated by injecting the vaccine intraperitoneally. After 8 weeks serum was sampled for measurement of antibodies against ISAV and the fish was challenged with live ISAV in a cohabitation infection model. A detailed description of the experiment is given in the following. ISA virus (Glesvaer/2/90) was grown in ASK cells, and the lysates of virus supernatant was inactivated by 0.1% formalin at room temperature for 3 days. After inactivation, formalin was removed by dialysis against PBS using Dispodialyzers at 4°C according to the instructions of the manufacturer (Spectrum Laboratories, CA). The virus inactivation was confirmed by inoculation on ASK cell in two subsequent passages. By measuring the antigenic content of the inactivated virus, the Hemagglutinin-esterase activity was measured by hemagglutination assay. Briefly, serial twofold dilutions of inactivated ISAV were prepared and incubated in quadruplet with 3% salmon erythrocytes in PBS at room temperature. After 60 min, the hemagglutination titer was measured as the reciprocal of the highest dilution producing complete hemagglutination. Hemagglutinin-Esterase Unit (HEU) of the inactivated virus was used in this study. Recombinant IFNa1 was prepared as described by Sun et al. (Sun B, Skjaeveland I, Svingerud T, Zou J, Jorgensen J, Robertsen B (2011). Antiviral activity of salmonid interferon gamma against infectious pancreatic necrosis virus and salmonid alphavirus and its dependency on type I interferon. Journal of Virology 85: 9188-9198). To produce recombinant IFNc (rIFNc), sub-confluent HEK293 cells were seeded in 24-well plates and transfected with 500 ng of IFNc expression plasmid using 7 µl FugeneHD Transfection Reagent (Roche Diagnostics) per well. The IFNc expression plasmid is described in Example 1. The medium was changed to EMEM with 10% FBS 5 h after transfection. Cell media containing IFNs were harvested 48 h after transfection, centrifuged for 5 min at 3000xg, filtrated through a 0.45 µm filter and frozen in aliquots at -70°C until use. IFNc activity was estimated to be 5800000 Units/ml measured as antiviral activity against infectious pancreatic necrosis virus as described by Berg et al (Berg K, Svingerud T, Sun B, Robertsen B. (2009). An antiserum against Atlantic salmon IFNal detects IFN and neutralizes antiviral activity produced by poly I:C stimulated cells. Developmental and Comparative Immunology 33, 638-45).

Each vaccine dose consisted of 0.1 ml contained 8 HEU inactivated ISAV (WVV) alone or combined with 100 ng rIFNa1 or 3.6x10⁴ Units IFNc with or without oil adjuvant. The vaccine was formulated as a water-in-oil emulsion using Montanide™ ISA 763A VG (Seppic) with an adjuvant/antigen ratio of 70/30 (weight/weight). Groups of Atlantic salmon presmolt (65 fish per group, average weight 30 gram) were anesthetized with 0.005% benzocaine and tagged by alcian blue group by group as described in Table 6.

**Table 6.**

| | **Vaccine** |
|---|---|
| Group 1 | oil adjuvant + HEK293 supernatant |
| Group 2 | oil adjuvant + WVV |
| Group 3 | oil adjuvant + WVV + HEK293 supernatant |
| Group 4 | oil adjuvant + rIFNa1 |
| Group 5 | oil adjuvant + WVV+ rIFNa1 |
| Group 6 | oil adjuvant + IFNc |
| Group 7 | oil adjuvant + WVV+ IFNc |
| Group 8 | WVV+ rIFNa1 |

Each fish was injected intraperitoneally with a vaccine dose of 0.1ml. All groups were kept in one 900 1 tank in fresh water of 10°C throughout the experiment. Group 1 was the control group, which was combined with the adjuvant and HEK293 cell supernatant. Group 2, 3, 5, and group 7 were all immunized with ISAV vaccine but with or without IFNs. Group 4 and 6 were injected with mock vaccine (adjuvant and IFN only). Group 8 was inactivated ISAV with rIFNa, but without oil adjuvant.

After 8 weeks post vaccination, serum was sampled from 15 fish before cohabitant infection, 104 shedder fish were injected i.p. with 0.1ml ISAV of the ISAV Glesvaer/2/90 isolate, approximately 10⁵ TCID₅₀/fish virus, and placed into the same tank. Mortality was recorded daily. The shedder fish started to die at day 15 post infection (p.i.), and the mortality of the vaccinated groups started at day 31 p.i. The experiment was ended at day 63 p.i. when the mortality of the control group had reached approximately 70%.

The development of cumulative mortality in each vaccine group is described in Fig. 5 and the final morality and relative percent survival of each vaccine group are shown in Table 7. The results show that recombinant IFNa1 and IFNc provides no protection against ISAV infection while inactivated ISAV in oil adjuvant gives approximately 50% protection. Addition of rIFNal to the WVV gave a somewhat smaller RPS of 37 % while addition of rIFNc to the WVV provided the best protection with an RPS of 65.7% demonstrating the adjuvant effect of rIFNc in the WVV.

Addition of IFNal to WVV without oil adjuvant gave an RPS of 40%, but this effect is difficult to interpret since WVV without oil and IFN was not included in the experiment.

**Table 7. Summary of cumulative mortality and relative percent survival (RPS) of salmon vaccinated with inactivated ISAV with or without recombinant IFNs after cohabitant challenge with ISAV.**

| | **Vaccine group** | **Cumulative Mortality (%)** | **RPS** |
|---|---|---|---|
| 1 | oil adjuvant + HEK293 supernatant | 70 | 0 |
| 2 | oil adjuvant + WVV | 34 | 51 |
| 3 | oil adjuvant + WVV + HEK293 supernatant | 36 | 48.5 |
| 4 | oil adjuvant + rIFNa1 | 68 | 2.9 |
| 5 | oil adjuvant + WVV+ rIFNa1 | 44 | 37 |
| 6 | oil adjuvant + IFNc | 66 | 5.7 |
| 7 | oil adjuvant + WVV+ IFNc | 24 | 65.7 |
| 8 | WVV+ rIFNa1 | 42 | 40 |

The antibody titers in each group are described in Fig. 6 and shows that WVV in oil adjuvant stimulates antibody production against ISAV after 8 weeks with an average titer of approximately 653 -710. However, addition of rIFNc to WVV gave a significantly higher average antibody titer of approximately 2268, which confirms the adjuvant effect of rIFNc in the WVV vaccine.

## Claims

1. An interferon coding sequence selected from the group consisting of SEQ ID NOs: 1, 3, and 4 for use in a method for vaccination as an adjuvant in combination with a DNA vaccine plasmid in salmon.

2. The interferon coding sequence for use according to claim 1, wherein the DNA vaccine plasmid is against a virus selected from the group consisting of infectious pancreatic necrosis virus (IPNV), salmonid alpha virus (SAV), viral hemorrhagic septicemia virus (VHSV), infectious haematopoietic necrosis virus (IHNV), infectious salmon anemia virus (ISAV), piscine reovirus (PRV), and piscine myocarditis virus (PMCV).

3. The interferon coding sequence for use according to claim 1, wherein the DNA vaccine plasmid is a polynucleotide sequence encoding hemagglutinin esterase from ISAV.

4. A plasmid comprising an interferon coding sequence selected from the group consisting of SEQ ID NOs: 1, 3, and 4 for use in a method for vaccination as an adjuvant in combination with a DNA vaccine plasmid in salmon, wherein the interferon coding sequence is linked to a promoter sequence.

5. The plasmid for use according to claim 4, wherein the plasmid further comprises a polynucleotide sequence encoding an antigen originates from a fish pathogen selected from the group consisting of IPNV, SAV, VHSV, IHNV, ISAV, PRV, and PMCV, and the antigen is operably linked to a promoter sequence.

6. The plasmid for use according to claim 5, wherein the polynucleotide sequence encodes hemagglutinin- esterase originating from ISAV.

7. A vaccine composition comprising:
(i) a DNA vaccine plasmid comprising a polynucleotide sequence encoding an antigen originating from a pathogen in salmon operably linked to a promoter; and
(ii) an interferon coding sequence selected from the group consisting of SEQ ID No. 1 encoding IFNa1, SEQ ID No. 3 encoding IFNb, and SEQ ID No. 4 encoding IFNc.

8. The vaccine composition according to claim 7, wherein the pathogen is selected from the group consisting of IPNV, SAV, VHSV, IHNV, ISAV, PRV, and PMCV.

9. The vaccine composition according to claim 8, wherein the antigen is hemagglutinin-esterase (HE) originating from ISAV.

10. The vaccine composition according to claim 7, wherein the interferon coding sequence (ii) is comprised in the DNA vaccine plasmid (i).

11. The vaccine composition according to claim 7, wherein the interferon coding sequence (ii) is present in a plasmid different from the DNA vaccine plasmid (i).

12. A recombinant IFNc (rIFNc) protein for use in a method for vaccination as an adjuvant in combination with an inactivated whole virus vaccine in salmon, wherein the recombinant IFNc (rIFNc) protein comprises a sequence of SEQ ID No. 8.

13. The recombinant IFNc for use according to claim 12, wherein the inactivated whole virus vaccine is against a virus selected from the group consisting of infectious pancreatic necrosis virus (IPNV), salmonid alpha virus (SAV), viral hemorrhagic septicemia virus (VHSV), Infectious haematopoietic necrosis virus (IHNV), infectious salmon anemia virus (ISAV), piscine reovirus (PRV), and piscine myocarditis virus (PMCV).

## Patentansprüche

1. Interferonkodierende Sequenz, ausgewählt aus der Gruppe bestehend aus SEQ ID Nr: 1, 3 und 4, zur Verwendung in einem Verfahren zur Impfung als ein Adjuvans in Kombination mit einem DNA-Impfstoffplasmid in Lachs.

2. Interferonkodierende Sequenz zur Verwendung nach Anspruch 1, wobei das DNA-Impfstoffplasmid gegen ein Virus ist, das aus der Gruppe bestehend aus infektiösem Pankreasnekrosevirus (IPNV), salmonidem Alphavirus (SAV), viralem hämorrhagischen Septikämievirus (VHSV), infektiösem hämatopoetischen Nekrosevirus (IHNV), Ansteckende Blutarmut der Lachsevirus (ISAV), Piscinem Reovirus (PRV) und Piscinem Myokarditisvirus (PMCV) ausgewählt ist.

3. Interferonkodierende Sequenz zur Verwendung nach Anspruch 1, wobei das DNA-Impfstoffplasmid eine Polynukleotidsequenz ist, die Hämagglutininesterase von ISAV kodiert.

4. Plasmid, umfassend eine interferonkodierende Sequenz, ausgewählt aus der Gruppe bestehend aus SEQ ID Nr: 1, 3 und 4, zur Verwendung in einem Verfahren zur Impfung als ein Adjuvans in Kombination mit einem DNA-Impfstoffplasmid in Lachs, wobei die interferonkodierende Sequenz mit einer Promotorsequenz verbunden ist.

5. Plasmid zur Verwendung nach Anspruch 4, wobei das Plasmid ferner eine Polynukleotidsequenz umfasst, die ein Antigen kodiert, das von einem Fischkrankheitserreger, ausgewählt aus der Gruppe bestehend aus IPNV, SAV, VHSV, IHNV, ISAV, PRV und PMCV, stammt, und das Antigen funktionsbereit mit einer Promotorsequenz verbunden ist.

6. Plasmid zur Verwendung nach Anspruch 5, wobei die Polynukleotidsequenz Hämagglutininesterase, stammend von ISAV, kodiert.

7. Impfstoffzusammensetzung, umfassend:
(i) ein DNA-Impfstoffplasmid, umfassend eine Polynukleotidsequenz, die ein Antigen kodiert, das aus einem Krankheitserreger in Lachs stammt, funktionsbereit verbunden mit einem Promotor; und
(ii) eine interferonkodierende Sequenz, ausgewählt aus der Gruppe bestehend aus SEQ ID Nr. 1, die IFNa1 kodiert, SEQ ID Nr. 3, die IFNb kodiert, und SEQ ID Nr. 4, die IFNc kodiert.

8. Impfstoffzusammensetzung nach Anspruch 7, wobei der Krankheitserreger aus der Gruppe bestehend aus IPNV, SAV, VHSV, IHNV, ISAV, PRV und PMCV ausgewählt ist.

9. Impfstoffzusammensetzung nach Anspruch 8, wobei das Antigen Hämagglutininesterase (HE), stammend von ISAV, ist.

10. Impfstoffzusammensetzung nach Anspruch 7, wobei die interferonkodierende Sequenz (ii) in dem DNA-Impfstoffplasmid (i) umfasst ist.

11. Impfstoffzusammensetzung nach Anspruch 7, wobei die interferonkodierende Sequenz (ii) in einem Plasmid vorhanden ist, das anders ist als das DNA-Impfstoffplasmid (i).

12. Rekombinantes IFNc (rIFNc) -Protein zur Verwendung in einem Verfahren zur Impfung als ein Adjuvans in Kombination mit einem inaktivierten Gesamtvirusimpfstoff in Lachs, wobei das rekombinante IFNc (rIFNc) -Protein eine Sequenz der SEQ ID Nr. 8 umfasst.

13. Rekombinantes IFNc zur Verwendung nach Anspruch 12, wobei der inaktivierte Gesamtvirusimpfstoff gegen ein Virus ist, das aus der Gruppe bestehend aus infektiösem Pankreasnekrosevirus (IPNV), salmonidem Alphavirus (SAV), viralem hämorrhagischen Septikämievirus (VHSV), infektiösem hämatopoetischem Nekrosevirus (IHNV), Ansteckende Blutarmut der Lachsevirus (ISAV), Piscinem Reovirus (PRV) und Piscinem Myokarditisvirus (PMCV) ausgewählt ist.

## Revendications

1. Séquence codant un interféron choisie dans le groupe constitué par SEQ ID NO: 1, 3 et 4, pour utilisation dans un procédé de vaccination en tant qu'adjuvant en combinaison avec un plasmide de vaccin à ADN chez le saumon.

2. Séquence codant un interféron pour utilisation selon la revendication 1, dans laquelle le plasmide de vaccin à ADN est contre un virus choisi dans le groupe constitué par un virus de nécrose pancréatique infectieuse (IPNV), un alphavirus des salmonidés (SAV), un virus de septicémie hémorragique virale (VHSV), un virus de nécrose hématopoïétique infectieuse (IHNV), un virus d'anémie infectieuse du saumon (ISAV), un réovirus pisciaire (PRV) et un virus de myocardite pisciaire (PMCV).

3. Séquence codant un interféron pour utilisation selon la revendication 1, dans laquelle le plasmide de vaccin à ADN est une séquence polynucléotidique codant l'hémagglutinine estérase de l'ISAV.

4. Plasmide comprenant une séquence codant un interféron choisie dans le groupe constitué par SEQ ID NO: 1, 3 et 4, pour utilisation dans un procédé de vaccination en tant qu'adjuvant en combinaison avec un plasmide de vaccin à ADN chez le saumon, dans laquelle la séquence codant un interféron est liée à une séquence promoteur.

5. Plasmide pour utilisation selon la revendication 4, lequel plasmide comprend en outre une séquence polynucléotidique codant un antigène provenant d'un pathogène de poisson choisi dans le groupe constitué par IPNV, SAV, VHSV, IHNV, ISAV, PRV et PMCV, et l'antigène est lié de façon opérationnelle à une séquence promoteur.

6. Plasmide pour utilisation selon la revendication 5, dans lequel la séquence polynucléotidique code une hémagglutinine-estérase provenant d'un ISAV.

7. Composition vaccinale comprenant :
(i) un plasmide de vaccin à ADN comprenant une séquence polynucléotidique codant un antigène provenant d'un pathogène du saumon lié de façon opérationnelle à un promoteur ; et
(ii) une séquence codant un interféron choisie dans le groupe constitué par SEQ ID NO : 1 codant IFNa1, SEQ ID NO : 3 codant IFNb, et SEQ ID NO : 4 codant IFNc.

8. Composition vaccinale selon la revendication 7, dans laquelle le pathogène est choisi dans le groupe constitué par IPNV, SAV, VHSV, IHNV, ISAV, PRV et PMCV.

9. Composition vaccinale selon la revendication 8, dans laquelle l'antigène est l'hémagglutinine-estérase (HE) provenant d'un ISAV.

10. Composition vaccinale selon la revendication 7, dans laquelle la séquence codant un interféron (ii) est comprise dans le plasmide de vaccin à ADN (i).

11. Composition vaccinale selon la revendication 7, dans laquelle la séquence codant un interféron (ii) est présente dans un plasmide différent du plasmide de vaccin à ADN (i).

12. Protéine IFNc recombinante (rIFNc), pour utilisation dans un procédé de vaccination en tant qu'adjuvant en combinaison avec un vaccin à virus entier inactivé chez le saumon, laquelle protéine IFNc recombinante (rIFNc) comprend une séquence de SEQ ID NO : 8.

13. IFNc recombinante pour utilisation selon la revendication 12, dans laquelle le vaccin à virus entier inactivé est contre un virus choisi dans le groupe constitué par un virus de nécrose pancréatique infectieuse (IPNV), un alphavirus des salmonidés (SAV), un virus de septicémie hémorragique virale (VHSV), un virus de nécrose hématopoïétique infectieuse (IHNV), un virus d'anémie infectieuse du saumon (ISAV), un réovirus pisciaire (PRV) et un virus de myocardite pisciaire (PMCV).


### SEQUENCE LISTING

<110> Robertsen, Børre
<120> The use of interferons as vaccine adjuvants
<130> P23036NO00
<160> 12
<170> PatentIn version 3.5
<210> 1
   <211> 829
   <212> DNA
   <213> Atlantic Salmon
<400> 1
   ggaaaactaa cagcgaaaca aacagctatt tacaatgtat acagtgcaga gttggacgtg       60
   tatttgtctt attatttgca gtatgcagag cgtgtgtcat tgctgtgact ggatccgaca      120
   ccactacggt cacttgagct cagaatacct ttccctgctg gaccagatgg gaggagatat      180
   cacaaagcag gatgccccag tctttttccc aacatccctt tacagacaca tagatgatgc      240
   cgaggttgag gaccaagtca gattcctgaa agagaccatc tatcaaatca caaaactgtt      300
   tgatgggaat atgaaatctg tcacctggga caagaaaaag ctggacgatt tcctcaacat      360
   tctagaacgc caattggaga accttaaatc ctgtgtatca cctgccatga aacctgagaa      420
   gagactgaaa cgctacttca agaagttgaa taagaatgtt ctgagaaaaa tgaactacag      480
   tgcacaggca tgggagctca tcaggaaaga gacaaaacgt catctgcaaa gattggatat      540
   ccttgcagca cagatgtact gatcatccag actcatttca gaagctacgc ccttaccagt      600
   ttataaagtt tgaatacaat cacgtgggac cctgtcagtc tatttattat atttaaatcc      660
   ataaattatt gttttttatt tgtattattt attatttata ttttgtattt cccttttgta      720
   cctgtgttga actgaactgt attgccggtc tactataaaa accaatgaaa aaataacatt      780
   gaaaatgtga ataaaaacgt attttgtata aaaaaaaaaa aaaaaaaaa                   829
<210> 2
   <211> 1290
   <212> DNA
   <213> Atlantic Salmon
<400> 2
   gaagactacg gaacaatatt tcggactaac tttgaacgaa acccactaaa ttaatgtcta        60
   atatggctac atttcttaag aagtgtgggt tttatttcga cttcgacgat tttgttatca       120
   gactcatttt atcggttcac gcgaagttat tagcagttga aagcaaagct cgcgaatggc       180
   cttttctcgc tatagtaata gtctagccga tatctaaatg gaataatctt atcttgtggt       240
   tcaactataa tttaaagctg atttcaagga gaatgtgtat agatttactg tgatttgttc       300
   gtccaggaga aggagcaggc atggaattca tactgctgca tgtgctagtc tggaaaatga       360
   aagttcccgc ctccggtagg ttgatcaggg gaaattctcc atacagtttt ctctttccaa       420
   attccacata aaactactta acacagccaa gatgttcaaa ctcatctgga aaactaacag       480
   cgaaacaaac tgctatttac aatgtataca atgcagagtt ggacgtgtat ttttcttatt       540
   atttgcagta tgcagagcgt gtatcattgc tgtgactgga tccgacacca ctacggtcac       600
   ttgagctcag aatacctttc cctgctggac cagatgggag gagatatcac aaagcaggat       660
   gccccagtct ttttcccaac atccctttac agacacatag atgatggcga ggttgaggac       720
   aaagtcagat tcctgaaaga gaccatctat caaatcacaa aactgtttga tgggaatatg       780
   aaatctgtca cctgggacaa gaaaaacctg gacgatttcc tcaacattct agaacgccaa       840
   ttggagaacc ttaattcctg tgtatcacct gccatgaaac ctgagaggag actgaaacgc       900
   tacttcaaga agttgaataa gaatgttctg agaaaaatga actacagtgc acaggcgtgg       960
   gagctcatca ggaaagagac aaaacgtcat ctgcaaagat tggatatcct tgcagcacag      1020
   atgtactgat catccagact catttcagaa gctacgccct taccagttta tagagcttga      1080
   atacaatcat gtgggaccct gtcagtctat ttattatatt taaatccata aattattgtt      1140
   ttttatttgt ataatttatt atttatatgt tgtatttccc ttttgtacct gtgttgaact      1200
   gaactgtatt gccggtctac tataaaaacc aatgaaaaaa ttatattcaa aatgtgaata      1260
   aaaacgtatt ttgtaaaaaa aaaaaaaaaa                                        1290
<210> 3
   <211> 553
   <212> DNA
   <213> Atlantic Salmon
<400> 3
   atggctgtat tgaaatggtt gagcatttgc ctgactctgt tctgccaagg cacagcagca 60
   tcaaaacctt gcaggtggac gcagtttagg ttggggaagc tgaacgatgt gagcataggc 120
   ctgctctcag atatgggtgg actctttcca cttatgtgtg cagaagaaag cgtcgaacaa 180
   atgtttccag aggatcttta caagaacaca gagggtgagg acgtctatgt ggtggcattg 240
   gaggctatgc gatatgtgga acaattatac aacaacagtc tgacgtctgt cacgtggaac 300
   aaaacaaaac ttaacatgtt ccaaaacgtc atatatcgtc aagttcaaaa cttagagtta 360
   tgtgtcgtag gtggtgtttg ggaatcctct ggagatggat ggtcggttac tctgaaaaca 420
   tacttcaaca agctgaacac cgtcttgaaa gagaaggaac acagcgcatg cgcatgggag 480
   attgtgcgaa aggagattcg cgaaaacttg gtgcagttca agaaattcat tgacagcaga 540
   gtcaagctgt gac 553
<210> 4
   <211> 565
   <212> DNA
   <213> Atlantic Salmon
<400> 4
   atggcacttc agactatcac ttggatgagc gccttcctct gcgtcgcgca cgtttgttcc 60
   atgcccatgc cttgccagct acaaggacag ctggtgcgaa taacccacaa cctactgaga 120
   gacatggggg gtaattttcc tctggagtgt cttcaggaga acgtctttgt ggcattccca 180
   gccaccgcat ttgcatcctc cggcgcgcca cagttgggca gcagtggtgc taaggctatt 240
   tatgagacat tgaagaacat cgacatattg tttgaagctg acgacctgcc tactcagtgg 300
   gaccaacaga agttgaagaa ttttcagaat attgtatacc gccagattga agaaagcaaa 360
   tgtatgatgg gcagtgtgga tacaagtgat tatctcatca ggacagaagg actgaatacg 420
   tactttggga acattgcagc agtcctaaaa gaaaagaatt tcagttactg cgcctgggaa 480
   gtggttcgaa aagagctcct gtacacccta cagttcattc tggaacacaa ctctgatagc 540
   cttctgtggg ccaacagaac atgac 565
<210> 5
   <211> 175
   <212> PRT
   <213> Atlantic salmon
<400> 5
   Met Tyr Thr Val Gln Ser Trp Thr Cys Ile Cys Leu Ile Ile Cys Ser
   1               5                   10                  15
   Met Gln Ser Val Cys His Cys Cys Asp Trp Ile Arg His His Tyr Gly
               20                  25                  30
   His Leu Ser Ser Glu Tyr Leu Ser Leu Leu Asp Gln Met Gly Gly Asp
           35                  40                  45
   Ile Thr Lys Gln Asp Ala Pro Val Phe Phe Pro Thr Ser Leu Tyr Arg
       50                  55                  60
   His Ile Asp Asp Ala Glu Val Glu Asp Gln Val Arg Phe Leu Lys Glu
   65                  70                  75                  80
   Thr Ile Tyr Gln Ile Thr Lys Leu Phe Asp Gly Asn Met Lys Ser Val
                   85                  90                  95
   Thr Trp Asp Lys Lys Lys Leu Asp Asp Phe Leu Asn Ile Leu Glu Arg
               100                 105                 110
   Gln Leu Glu Asn Leu Lys Ser Cys Val Ser Pro Ala Met Lys Pro Glu
           115                 120                 125
   Lys Arg Leu Lys Arg Tyr Phe Lys Lys Leu Asn Lys Asn Val Leu Arg
       130                 135                 140
   Lys Met Asn Tyr Ser Ala Gln Ala Trp Glu Leu Ile Arg Lys Glu Thr
   145                 150                 155                 160
   Lys Arg His Leu Gln Arg Leu Asp Ile Leu Ala Ala Gln Met Tyr
                   165                 170                 175
<210> 6
   <211> 175
   <212> PRT
   <213> Atlantic Salmon
<400> 6
   Met Tyr Thr Met Gln Ser Trp Thr Cys Ile Phe Leu Ile Ile Cys Ser
   1               5                   10                  15
   Met Gln Ser Val Tyr His Cys Cys Asp Trp Ile Arg His His Tyr Gly
               20                  25                  30
   His Leu Ser Ser Glu Tyr Leu Ser Leu Leu Asp Gln Met Gly Gly Asp
           35                  40                  45
   Ile Thr Lys Gln Asp Ala Pro Val Phe Phe Pro Thr Ser Leu Tyr Arg
       50                  55                  60
   His Ile Asp Asp Gly Glu Val Glu Asp Lys Val Arg Phe Leu Lys Glu
   65                  70                  75                  80
   Thr Ile Tyr Gln Ile Thr Lys Leu Phe Asp Gly Asn Met Lys Ser Val
                   85                  90                  95
   Thr Trp Asp Lys Lys Asn Leu Asp Asp Phe Leu Asn Ile Leu Glu Arg
               100                 105                 110
   Gln Leu Glu Asn Leu Asn Ser Cys Val Ser Pro Ala Met Lys Pro Glu
           115                 120                 125
   Arg Arg Leu Lys Arg Tyr Phe Lys Lys Leu Asn Lys Asn Val Leu Arg
       130                 135                 140
   Lys Met Asn Tyr Ser Ala Gln Ala Trp Glu Leu Ile Arg Lys Glu Thr
   145                 150                 155                 160
   Lys Arg His Leu Gln Arg Leu Asp Ile Leu Ala Ala Gln Met Tyr
                   165                 170                 175
<210> 7
   <211> 183
   <212> PRT
   <213> Atlantic salmon
<400> 7
   Met Ala Val Leu Lys Trp Leu Ser Ile Cys Leu Thr Leu Phe Cys Gln
   1               5                   10                  15
   Gly Thr Ala Ala Ser Lys Pro Cys Arg Trp Thr Gln Phe Arg Leu Gly
               20                  25                  30
   Lys Leu Asn Asp Val Ser Ile Gly Leu Leu Ser Asp Met Gly Gly Leu
           35                  40                  45
   Phe Pro Leu Met Cys Ala Glu Glu Ser Val Glu Gln Met Phe Pro Glu
       50                  55                  60
   Asp Leu Tyr Lys Asn Thr Glu Gly Glu Asp Val Tyr Val Val Ala Leu
   65                  70                  75                  80
   Glu Ala Met Arg Tyr Val Glu Gln Leu Tyr Asn Asn Ser Leu Thr Ser
                   85                  90                  95
   Val Thr Trp Asn Lys Thr Lys Leu Asn Met Phe Gln Asn Val Ile Tyr
               100                 105                 110
   Arg Gln Val Gln Asn Leu Glu Leu Cys Val Val Gly Gly Val Trp Glu
           115                 120                 125
   Ser Ser Gly Asp Gly Trp Ser Val Thr Leu Lys Thr Tyr Phe Asn Lys
       130                 135                 140
   Leu Asn Thr Val Leu Lys Glu Lys Glu His Ser Ala Cys Ala Trp Glu
   145                 150                 155                 160
   Ile Val Arg Lys Glu Ile Arg Glu Asn Leu Val Gln Phe Lys Lys Phe
                   165                 170                 175
   Ile Asp Ser Arg Val Lys Leu
               180
<210> 8
   <211> 183
   <212> PRT
   <213> Atlantic salmon
<400> 8
   Met Ala Val Leu Lys Trp Leu Ser Ile Cys Leu Thr Leu Phe Cys Gln
   1               5                   10                  15
   Gly Thr Ala Ala Ser Lys Pro Cys Arg Trp Thr Gln Phe Arg Leu Gly
               20                  25                  30
   Lys Leu Asn Asp Val Ser Ile Gly Leu Leu Ser Asp Met Gly Gly Leu
           35                  40                  45
   Phe Pro Leu Met Cys Ala Glu Glu Ser Val Glu Gln Met Phe Pro Glu
       50                  55                  60
   Asp Leu Tyr Lys Asn Thr Glu Gly Glu Asp Val Tyr Val Val Ala Leu
   65                  70                  75                  80
   Glu Ala Met Arg Tyr Val Glu Gln Leu Tyr Asn Asn Ser Leu Thr Ser
                   85                  90                  95
   Val Thr Trp Asn Lys Thr Lys Leu Asn Met Phe Gln Asn Val Ile Tyr
               100                 105                 110
   Arg Gln Val Gln Asn Leu Glu Leu Cys Val Val Gly Gly Val Trp Glu
           115                 120                 125
   Ser Ser Gly Asp Gly Trp Ser Val Thr Leu Lys Thr Tyr Phe Asn Lys
       130                 135                 140
   Leu Asn Thr Val Leu Lys Glu Lys Glu His Ser Ala Cys Ala Trp Glu
   145                 150                 155                 160
   Ile Val Arg Lys Glu Ile Arg Glu Asn Leu Val Gln Phe Lys Lys Phe
                   165                 170                 175
   Ile Asp Ser Arg Val Lys Leu
               180
<210> 9
   <211> 26
   <212> DNA
   <213> Atlantic salmon
<400> 9
   aacatggctg tattgaaatg gttgag 26
<210> 10
   <211> 25
   <212> DNA
   <213> Atlantic salmon
<400> 10
   tcacagcttg actctgctgt caatg 25
<210> 11
   <211> 27
   <212> DNA
   <213> Atlantic salmon
<400> 11
   agaatggcac ttcagactat cacttgg 27
<210> 12
   <211> 25
   <212> DNA
   <213> Atlantic salmon
<400> 12
   tcatgttctg ttggcccaca gaagg 25